# EUROPEAN PATENT SPECIFICATION

(11) **EP 1 724 340 B1**
(45) Date of publication and mention of the grant of the patent: **18.02.2009**
(21) Application number: 05010216.9
(22) Date of filing: 11.05.2005
(51) Int. Cl.: C12N 15/01, C12R 1/01, A23L 1/30

(54) **Antibiotic-sensitive lactic acid bacteria strains**
Antibiotikasensitive Milchsäurebakterienstämme
Souches de bactéries lactiques sensitives aux antibiotiques

(43) Date of publication of application: 22.11.2006
(73) Proprietor: Chr. Hansen A/S, 2970 Hoersholm (DK)
(72) Inventor: Strøman, Per, 2850 Nærum (DK)
(74) Representative: Hagen, Klaus Bach

(56) References cited:
- WO-A-99/10476
- WO-A-03/099037
- MOUBARECK C ET AL: "Antimicrobial susceptibility of bifidobacteria." THE JOURNAL OF ANTIMICROBIAL CHEMOTHERAPY. JAN 2005, vol. 55, no. 1, January 2005 (2005-01), pages 38-44, XP002346517 ISSN: 0305-7453
- DELGADO S ET AL: "Antibiotic susceptibility of Lactobacillus and Bifidobacterium species from the human gastrointestinal tract" CURRENT MICROBIOLOGY, vol. 50, no. 4, April 2005 (2005-04), pages 202-207, XP002346518 ISSN: 0343-8651
- SCOTT K P ET AL: "Occurrence of the new tetracycline resistance gene tet(W) in bacteria from the human gut." ANTIMICROBIAL AGENTS AND CHEMOTHERAPY, vol. 44, no. 3, March 2000 (2000-03), pages 775-777, XP002346519 ISSN: 0066-4804
- VON WRIGHT A: "Regulating the safety of probiotics - The European approach" CURRENT PHARMACEUTICAL DESIGN, vol. 11, no. 1, 2005, pages 17-23, XP008053009 ISSN: 1381-6128
- ZHOU J S ET AL: "Antibiotic susceptibility profiles of new probiotic Lactobacillus and Bifidobacterium strains" INTERNATIONAL JOURNAL OF FOOD MICROBIOLOGY, vol. 98, no. 2, 1 February 2005 (2005-02-01), pages 211-217, XP004727760 ISSN: 0168-1605
- KHEADR E ET AL: "Comparison of the sensitivity of commercial strains and infant isolates of bifidobacteria to antibiotics and bacteriocins" INTERNATIONAL DAIRY JOURNAL, vol. 14, no. 12, December 2004 (2004-12), pages 1041-1053, XP004573489 ISSN: 0958-6946
- TEMMERMAN R ET AL: "Identification and antibiotic susceptibility of bacterial isolates from probiotic products." INTERNATIONAL JOURNAL OF FOOD MICROBIOLOGY, vol. 81, no. 1, 25 February 2003 (2003-02-25), pages 1-10, XP002346520 ISSN: 0168-1605
- CHARTERIS W P ET AL: "Antibiotic susceptibility of potentially probiotic Bifidobacterium isolates from the human gastrointestinal tract" LETTERS IN APPLIED MICROBIOLOGY, vol. 26, no. 5, May 1998 (1998-05), pages 333-337, XP000929181
- LIM K S ET AL: "Antimicrobial susceptibility of bifidobacteria." JOURNAL OF DAIRY SCIENCE, vol. 76, no. 8, August 1993 (1993-08), pages 2168-2174, XP002346521 ISSN: 0022-0302
- MATTEUZZI D ET AL: "Antimicrobial susceptibility of Bifidobacterium." ANNALES DE MICROBIOLOGIE, vol. 134A, no. 3, May 1983 (1983-05), pages 339-349, XP008052994 ISSN: 0300-5410
- CHOPRA I ET AL: "Tetracycline antibiotics: mode of action, applications, molecular biology, and epidemiology of bacterial resistance." MICROBIOLOGY AND MOLECULAR BIOLOGY REVIEWS, vol. 65, no. 2, June 2001 (2001-06), pages 232-260 ; sec, XP002346522 ISSN: 1092-2172
- CHARTERIS W P ET AL: "Gradient diffusion antibiotic susceptibility testing of potentially probiotic lactobacilli." JOURNAL OF FOOD PROTECTION, vol. 64, no. 12, December 2001 (2001-12), pages 2007-2014, XP009006815 ISSN: 0362-028X
- TANAKA H ET AL: "Bile salt hydrolase of Bifidobacterium longum-biochemical and genetic characterization." APPLIED AND ENVIRONMENTAL MICROBIOLOGY, vol. 66, no. 6, June 2000 (2000-06), pages 2502-2512, XP002346523 ISSN: 0099-2240
- PIATKIEWICZ A ET AL: "Influence of UV-rays and nitrosoguanidine on the proteolytic activity of Lactobacillus casei" ACTA ALIMENTARIA POLONICA, vol. 4, no. 2, 1978, pages 217-228, XP008053017 ISSN: 0137-1495
- AKCELIK M ET AL: "Definition of genetic determinants of nisin production ability in Lactococcus lactis subsp. lactis strains isolated from Turkey" TURKISH JOURNAL OF BIOLOGY, vol. 20, no. SUPPL., 1996, pages 9-18, XP008053015 ISSN: 1300-0152
- CHOPRA I ET AL: "Tetracycline antibiotics: mode of action, applications, molecular biology, and epidemiology of bacterial resistance" MICROBIOLOGY AND MOLECULAR BIOLOGY REVIEWS, vol. 65, no. 2, June 2001 (2001-06), pages 232-260,

## Description

### FIELD OF THE INVENTION

The present invention pertains to a method of obtaining novel antibiotic-sensitive strains of the genus of *Bifidobacterium* from antibiotic-resistant *Bifidobacteriaceae*carrying a chromosomal encoded antibiotic resistance gene and the strains obtainable by the method. In particular, the present invention relates to novel antibiotic-sensitive strains obtained from antibiotic-resistant probiotic strains and the use of such novel strains for the preparation of a food or feed product or a dosage form comprising viable organisms.

### BACKGROUND OF THE INVENTION

Bacteria which ferment sugars with the production of acids in particular lactic acid as a major metabolic component has been known for a long time. Such bacteria may be found in milk or milk products, living or decaying plants but also in the intestine of man and animals. Traditionally, these bacteria have been referred to as "lactic acid bacteria". Lactic acid bacteria designates a rather heterologous group of Gram positive, non-motile, microaerophilic or anaerobic bacteria which ferment sugar with the production of acids including lactic acid and comprise e.g. the genera *Bifidobacterium, Enterococcus, Lactobacillus, Lactococcus,* Leuconostoc and Pediococcus.

For centuries lactic acid bacteria have been used in the manufacture of food and feed products including most dairy products and today lactic acid bacteria are essential in the making of all fermented milk products such as yoghurt, cheese and butter. Furthermore lactic acid bacteria are widely used in the meat processing industry, wine manufacturing industry, the juice manufacturing industry as well as a number of other industries.

Cultures of lactic acid bacteria also find important uses in the biopreservation of foodstuffs.

The publication of a large amount of reports documenting that various lactic bacteria beneficially affect the well-being of humans and/or animals have attracted even further interest to this group of bacteria. In particular, specific strains of *Lactobacillus* or *Bifidobacterium* have been found to be able to colonize the intestinal mucosa and to assist in the maintenance of the well-being of the hosts.

EP 0 768 375 describes specific strains of *Bifidobacterium* sp, that are capable of becoming implanted in the intestinal flora and being capable of competitively excluding adhesion of pathogenic bacteria to intestinal cells. These *Bifidobacteria* are reported to assist in immunomodulation and thus in the maintenance of the individual's health. The immunomodulation effect of *Bifidobacteria* may even be conferred onto unborn children. WO 01/97822 e.g. describes that intake of *Bifidobacterium animalis* strain Bb-12® by the mother during her pregnancy reduces the occurrence of atophic diseases in children. Also WO 03/099037 describes that *Bifidobacterium animalis* strain BB-12® is able to beneficially modify the immune response. According to Masco et al. (2004) *Bifidobacterium animalis* strain BB-12® should correctly be referred to as *Bifidobacterium animalis* subsp. lactis strain BB-12®.

Probiotic microorganisms have been defined as "Live microorganisms which when administered in adequate amounts confer a health benefit on the host" (FAO / WHO 2002). During the recent years, documentation on probiotic properties of *Bifidobacteria* and other lactic bacteria have accumulated. In general the probiotic activity is associated with specific strains. The previously mentioned *Bifidobacterium animalis* strain BB-12® as well as *Bifidobacterium lactis* strain HN019 have been reported as probiotic (WO 01/97822, WO 03/099037, Zhou et al. (2005), US 6379663).

Worldwide there is widespread public concern that the number of antibiotic resistant pathogenic bacteria increase dramatically. All available data indicate that the disturbing increase in antibiotic resistant pathogenic bacteria is caused by an extensive and very liberal use of antibiotics in the general population as well as in animal husbandry.

It is a well established fact that many antibiotic resistant bacteria carry genetic determinants, genes, which confer resistance to one or more antibiotics It is furthermore well known that such genetic determinants under certain circumstances are transferable and may confer the antibiotic-resistant phenotype to recipient bacteria. The frequency of transfer is very much dependent on the particular genetic context in which the antibiotic resistance genes are found. I.e. antibiotic-resistant genes residing on plasmids or on transposons have been demonstrated to confer the antibiotic-resistant phenotype to recipient bacteria at relatively high frequencies, whereas chromosomally encoded determinants are very much less prone to move.

For these reasons it may be of concern to ingest even beneficially, non-pathogenic bacteria if they do contain an antibiotic resistant determinant. This concern is further emphasized in the report from the EUROPEAN COMMISSION's Scientific Committee on Animal Nutrition (SCAN) on the Criteria for Assessing the Safety of Micro-Organisms Resistant to Antibiotics of Human Clinical and Veterinary of 3 July 2001, revised on 24 January 2003, stating that the presence of a known resistance gene is not acceptable (page 21).

Resistance to tetracycline is the most common bacterial antibiotic resistance found in nature and similarly is the most widely distributed type of resistance among bacteria isolated from animals (Billington 2002). Tetracycline inhibits protein synthesis by binding to a single high-affinity site on the 30S ribosomal subunit. With tetracycline in this position, the binding of aminoacyl-tRNA to the A site is prevented and thus protein synthesis is blocked.

Resistance to tetracycline may be mediated either by active efflux of tetracycline from the cell, by ribosomal protection by one or more soluble protein(s), the so-called ribosomal protection proteins (RPPs), or by enzymatic inactivation of tetracycline.

Recently, a new ribosome-protection-type tetracycline resistance (Tet') gene, *tetW,* was identified in rumen isolates of *Butyrivibrio fibrisolvens* and a number of other rumen bacteria (Barbosa, 1999).

Although the *tetW* determinant is widely distributed among tetracycline resistant isolates of animal pathogens (Billington 2002) it was a surprise for the authors of this application to find that all known probiotic strains of *Bifidobacterium animalis* subs. *lactis,* including the two well-known *Bifidobacterium* strains BB-12® and DR10^{™}, carry a functional *tetW* determinant and are resistant to tetracycline; in particular because the DR10^{™} strain as well as the BB-12® strain were reported to be tetracycline sensitive in a recent report (Zhou et al. 2005).

Even though, extensive experiments have indicated that the *tetW* determinant of *Bifidobacterium animalis* subspecies *lactis* strain BB-12® not is movable under realistic situations the concern of antibiotic resistant determinants in our food products still remains. Consequently, we have attempted several approaches to accomplish inactivation or removal of the *tetW* gene in *Bifidobacterium animalis subspecies lactis* BB-12® by classical mutagenesis, involving various mutagens, as well as by direct genetic manipulation. However, until now all attempts have been unsuccessful. It is contemplated that an important reason to the many unsuccessful attempts is the fact that the tetW is located on the chromosome of probiotic *Bifidobacterium animalis* subsp. *lactis* strains.

Thus it would be highly advantageous to establish a method for the inactivation of the *tetW* resistance gene in probiotic *Bifidobacteriaceae.* Such method could furthermore help to solve the problem of providing antibiotic sensitive variants of commercial interesting probiotic, tetracycline resistant *Bifidobacteriaceae,* that may meet the requirement of absence of functional antibiotic resistance genes.

### SUMMARY OF THE INVENTION

The above problem has been solved by providing a method of isolating a tetracycline sensitive strain of *Bifidobacterium* sp. (*Bifidobacteriaceae*) from a tetracycline-resistant bacterial progenitor strain wherein the antibiotic resistant phenotype is caused by the expression of a functional *tetW* that is stably integrated in its chromosome. Said method comprising subjecting the cells both to a chemical mutagen and a physical mutagen. The method comprises the steps of: 1) culture the progenitor cells to obtain a culture of exponential growing cells, 2) transfer an aliquot of the cells to fresh medium containing ethidium bromide (EtBr), 3) transfer the culture to one or more containers to form a 0.5 -10 mm thick layer of culture, 4) subject the culture to a UV treatment, 5) culture the mutated cells to obtain a culture of exponential growing cells, 6) transfer an aliquot of bacteria to one or more petridishes containing a suitable agar growth medium, the aliquot of bacteria is selected to give single colonies, 7) identify those colonies that have acquired antibiotic sensitivity by replica plating to petridishes with and without tetracycline antibiotic, and 8) isolate, expand and keep those antibiotic sensitive identified as a new tetracycline antibiotic sensitive strain.

This method appears general applicable to tetracycline resistant *Bifidobacterium* sp. with a functional *tetW* stably integrated in their chromosome, as we in only two attempts were able to isolate tetracycline sensitive variants of the two *Bifidobacterium* strains BB-12® and HN019 (DR10^{™}).

Thus further important aspects of the invention is the provision of strains of *Bifidobacterium* sp. containing an inactivating mutation in chromosomally encoded *tetW* rendering the strain sensitive to tetracyclines which is obtainable by the above mentioned method of, and the use of such *Bifidobacterium* strains for the preparation of an ingestible material or a medicament.

### DETAILED DISCLOSURE OF THE INVENTION

It is a primary result of the present invention that the inventor is able to disclose a generally useful method for obtaining new strains of *Bifidobacterium* sp. that contains a mutated *tetW* on its chromosome which renders the strain sensitive to tetracyclines and which is isolated from a tetracycline-resistant bacterial progenitor strain wherein the antibiotic resistant phenotype is caused by the expression of *tetW* stably integrated its chromosome.

In the art dilution tests are used to determine the minimum inhibitory concentrations (MICs) of antimicrobial agents and are the reference methods for antimicrobial susceptibility testing. In dilutions tests, microorganisms are tested for their ability to produce visible growth in suitable media and the lowest concentration of an antimicrobial agent that inhibits the growth of a microorganism is defined the MIC.

By the expression "tetracycline-resistant" is referred to a bacterium which have a minimum inhibitive concentration (MIC) of tetracycline of at least 5 microgram/ml, such as at least 8 microgram/ml including at least 10 microgram/ml or even at least 15 microgram tetracycline/ml, as determined by the "Etest" susceptibility screening method as described by Danielsen and Wind (2003). The minimum inhibitive concentration is the lowest concentration that results in inhibition of visible growth.

In the present context the expression "sensitive to tetracycline" refer to a bacterium which have an MIC of 1.5 microgram tetracycline/ml or less, such as 1 microgram/ml or even less than 0.75 microgram tetracycline/ml, as determined by the "Etest" susceptibility screening method.

By "tetracyclines" or "tetracycline group of antibiotics is referred to the group af bactiostatic antibiotics that are produced by *Streptomyces* species, and their related semisynthetic derivatives. Tetracyclines inhibit both Gram-positive and Gram-negative bacteria and rickettsiae. They are characterized by a mode of action which imply that the antibiotic reversibly bind to the 30S ribosome and inhibit binding of aminoacyl-t-RNA to the acceptor site on the 70S ribosome. In addition to tetracycline itself also terramycin, demeclocycline, meclocycline, doxycycline / doxycyclin, lymecycline, methacycline, minocycline, oxytetracycline, rolitetracycline, aureomycin as well as other chlortetracyclines are considered as members of the group of tetracyclines. Consequently also a method, wherein tetracyclines is a antibiotic selected from the group of tetracycline, terramycin, demeclo-cycline, meclocycline, doxycycline / doxycyclin, lymecycline, methacycline, minocycline, oxytetracycline, rolitetracycline, aureomycin and other chlortetracyclines is an embodiment of the present invention.

As mentioned it required several attempts before the present method was developed. It is contemplated that the combined action of both a chemical and a physical mutagen, i.e. ethidium bromide and ultraviolet light, is essential for the successful mutation of the *tetW* gene.

To further increase the likelihood of success the culture may subsequent to the mutation step be subjected to an enrichment step for mutations comprising the steps of: a) transfer an aliquot of the UV treated culture to fresh medium containing a dose of a penicillin analogue which is detrimental to exponentially growing cells, but tolerable to non-growing cells, and b) culture the cells in said penicillin analogue comprising medium under conditions which would promote exponential growth in the absence of a penicillin analogue.

Considering that such an "ampicillin selection procedure" is well established and has been frequently used for gram-negative bacteria since 1972 (Miller 1972), it is surprising that the "ampicillin selection procedure" to the best of our knowledge not before has been used in a mutation protocol directed against gram-positive *Bifidobacteriaceae.*

Although some *Bifidobacteriaceae* may grow under aerobic conditions it is considered advantageous when the culture is performed at a reduced oxygen tension, e.g. by supplying the growth medium with cysteine hydrochloride as described in example 1.

In general, the dual mutagenic approach of the present invention is considered a more forceful scale than when the mutagens are used individually. As illustrated in example 1 the combined action of EtBr and exposure to UV light reduces the viability of the cells immediately after the EtBr-UV treatments considerably. It is contemplated that such forceful dual approach is essential for achieving effective inactivation of tetW in Bifidobacteriace. Thus in one important embodiment of the present invention the UV-treatment is adjusted to result in a reduction of the number of living cells measured by Colony Forming Units (CFUs) to less than 20%, such as less than 15% or even less than 10% relative to the number of the CFUs of the culture immediately before the UV-treatment.

In a preferred embodiment the EtBr concentration is adjusted to be between 10 and 30 microgram/ml, and in further embodiments the penicillin analogue used in the "ampicillin enrichment procedure" is amphicillin being used at a dose of 50-300 microgram/ml in the medium.

As Bifidobacteria in general are considered as probiotic organisms (Yazid, 2000) it should be noted that resistance to tetracyclines is widely distributed amongst Bifidobacteria.

Resistance to tetracycline and oxytetracycline has been observed in strains of *Bifidobacterium catenulatum, Bifidobacterium longum, Bifidobacterium pseudocatenulatum, Bifidobacterium asteroids, Bifidobacterium infantis, Bifidobacterium lactis, Bifidobacterium animalis* and subspecies thereof (Yazid (2000), Lim (1983), Scott (2000), this study). Importantly, *tetW* has been observed in tetracycline resistant strains of *Bifidobacterium longum, Bifidobacterium pseudocatenulatum, Bifidobacterium bifidum, Bifidobacterium lactis, Bifidobacterium animalis* and subspecies thereof (Scott et al. 2000, Moubareck et al. 2005, this study).

Thus in presently preferred embodiments, the bacterial species is selected from the group consisting of *Bifidobacteriacea* that contains a functional tetW rendering the bacteria resistant to tetracycline.

Useful *Bifidobacterium* species include *Bifidobacterium adolescentis, Bifidobacterium animalis, Bifidobacterium asteroids, Bifidobacterium bifidum, Bifidobacterium breve, Bifidobacterium catenulatum, Bifidobacterium infantis, Bifidobacterium lactis, Bifdobacterium longum and Bifidobacterium pseudocatenulatum,*

The invention is not, however, limited to these above mentioned particular Bifidobacteriacea. The person skilled in the art would recognize those Bifidobacteriacea which may be useful in the method according to the invention, as well as other probiotic bacteria which contain a functional *tetW* rendering them resistant to tetracyclines.

In the preferred embodiment of the method the progenitor strain is a strain of *Bifidobacterium animalis* subspecies *lactis.* In particular a method, wherein the progenitor strain is *Bifidobacterium animalis* subspecies *lactis* strain CHCC5445 (BB-12®), deposited on September 30 , 2003 according to the Budapest Treaty on the International Recognition of the Deposit of Microorganisms for the Purposes of Patent Procedure with the Deutsche Sammlung von Mikroorganismen und Zellkulturen under accession number DSM15954 or wherein the progenitor strain is *Bifidobacterium animalis* subspecies *lactis* strain CHCC7158, deposited on April 28 , 2005 according to the Budapest Treaty on the International Recognition of the Deposit of Microorganisms for the Purposes of Patent Procedure with the Deutsche Sammlung von Mikroorganismen und Zellkulturen under accession number DSM17280.

It is noteworthy to emphasize that the nomenclature of *Bifidobacterium animalis* subspecies *lactis* has changed over the years.

Initially the *Bifidobacterium* BB-12® strain was described as a *Bifidobacterium bifidum.* Subsequently it was found that *Bifidobacterium animalis* was more correct, although the strain is not a typical *Bifidobacterium animalis.* The strain differs in several aspects from *Bifidobacterium animalis* as described by Meile et al (1997), who suggested to establish a new species, *Bifidobacterium lactis.* This species name was later validated on list no. 62 in IJSB (1997). The species status of *Bifidobacterium lactis* has been discussed since Meiles publication. Recently, Cai et al. (2000) published DNA-DNA hybridisation results, which showed that *Bifidobacterium lactis* did not differ enough from *Bifidobacterium animalis* to allow species status. Based on these results the International Committee on Systematic Bacteriology, Subcommittee on the taxonomy of *Bifidobacterium, Lactobacillus* and related organisms has decided that *Bifidobacterium lactis* cannot be acknowledged as a valid species (Minutes, IJSEM, 2001). Since then a polyphasic taxonomic analysis has been done and published leading to the creation of two subspecies within *Bifidobacterium animalis* (Masco et aL, 2004), BB-12® belongs to one of the subspecies, B. *animalis* subsp. *lactis*. Based on DNA fingerprints it appears to us that also the well-known *Bifidobacterium* strain DR10^{™} correctly should be designated as B. *animalis* subsp. *lactis.* In the literature strain DR10^{™}, is also referred to as *Bifidobacterium lactis* HN019 (Zhou 2005) and HOWARU^{™} Bifido (www.danisco.com).

As illustrated in example 2, the method of the present invention may result in two classes of novel antibiotic-sensitive isolates one class which express an intermediate level of tetracycline sensitivity, i.e. isolates with an MIC ranging between 2 and 4 µg tetracycline/ml, and isolates with an MIC lower than 1.5 µg tetracycline/ml such as 0.75 or even 0.5 µg tetracycline/ml. Until now we have only identified isolates with an inactivated *tetW* in isolates with an MIC lower than 1.5 µg tetracycline/ml, and in all cases we used *Bifidobacteriacea* having an MIC larger than 10 microgram tetracycline/ml as progenitor cells. Thus in a preferred embodiment of the present invention is a method of isolating a tetracycline sensitive strain of *Bifidobacterium* sp. (*Bifidobacteriacea*) from a tetracycline-resistant bacterial progenitor strain wherein the Minimum inhibitive Concentration (MIC) of tetracycline of the progenitor strain is at least 10 microgram tetracycline/ml and the MIC of the antibiotic sensitive strain is 1 microgram tetracycline /ml or less.

As the tetracycline group of antibiotics share the same mode of action it is contemplated that the inactivation of *tetW* in general will result in a sensitivity shift to any of the tetracycline group of antibiotics of a size similar to the shift observed with tetracycline. Consequently an embodiment of the invention is a method of isolating a strain of *Bifidobacterium* sp. *(Bifidobacteriacea)* that is sensitive to one or more of tetracyclines from a bacterial progenitor strain that is resistant to one or more of the tetracyclines and wherein the Minimum inhibitive Concentration (MIC) of said antibiotic of the progenitor strain is at least 10-fold higher than the MIC of the antibiotic sensitive strain.

Examples of the tetracycline group of antibiotics are represented by the group of antibiotics comprising tetracycline, terramycin, demeclocycline, meclocycline, doxycycline /doxycyclin, lymecycline, methacycline, minocycline, oxytetracycline, rolitetracycline, aureomycin and other chlortetracyclines.

To the best of our knowledge until now all probiotic strains of *Bifidobacterium animalis* subsp. *lactis,* and a substantial number of other *Bifidobacteriaceae* carry a functional *tetW* determinant rendering them resistant to tetracycline. The inventor of the present invention surprisingly discovered that it was possible to provide *Bifidobacteriaceae* that carry an inactivated *tetW* determinant.

As illustrated in the examples it is indeed possible to obtain variants of known probiotic strains of *Bifidobacterium* sp. that contains a mutated, chromosomally encoded *tetW* rendering the strain sensitive to tetracyclines. The provision of such new strains is considered the most preferred embodiment of the present invention.

The new strain may in principle be a variant or mutation of any *Bifidobacterium* that carry a chromosomally encoded *tetW* rendering the strain resistant to tetracyclines. Suitable cells may be selected from the group of *Bifidobacteriacea* comprising *Bifidobacterium longum, Bifidobacterium pseudocatenulatum, Bifidobacterium bifidum, Bifidobacterium lactis, Bifidobacterium animalis* and subspecies thereof. In particular cells classified as *Bifidobacterium animalis* subspecies *lactis* are preferred.

In general new bacterial strains having a Minimum inhibitive Concentration (MIC) of antibiotic which is at least 10-fold lower than the MIC of the antibiotic resistant progenitor strain are preferred.

Preferred strains are those having an MIC that is 1.5 microgram tetracycline/ml or less, such as 1 microgram/ml or even less than 0.75 microgram tetracycline/ml as determined by the "Etest" susceptibility screening method.

Such strains which harbor a mutated *tetW* are particularly preferred embodiments of the invention.

The inactivating mutation of the *tetW* gene that renders the new strains sensitive to tetracyclines may typically be described in relation to the *tetW gene* sequence of the relevant progenitor cell.

As described in the examples suitable tetracycline-sensitive strains with an inactivated *tetW* can be achieved by introducing specific mutations in the *tetW* gene.

In one preferred embodiment of the invention an "opal" stop codon has been introduced in the tetW by changing part of chromosomally encoded tetW that is characterized by the sequence TCG CTG GGA TAC TTG AAC CAG AGT [SEQ ID 1] to TCG CTG GGA TAC TGA ACC AGA GTT [SEQ ID 2], the deleted base in the functional tetW is indicated by underscoring. Thus a Bifidobacterium which comprise the sequence: GGA TAC TGA ACC [SEQ ID 3] in its *tetW* gene is a preferred embodiment of the present invention. We also observed that the tetW gene could be inactivated, and result in sensitivity to tetracycline, by changing the part of chromosomally encoded tetW which comprise the sequence: CAG AGC GTG GTT CAG TCT GTT CGG [SEQ ID 4] to CAG AGC GTG GTT TAG TCT GTT CGG [SEQ ID 5] this mutation introduce an "amber" stop codon in tetW and the mutated base in the functional tetW is underscored. Such a Bifidobacterium which comprise the sequence: GTG GTT TAG TCT [SEQ ID 6] in its *tetW* gene is another preferred embodiment of the present invention. A bacterial strain wherein the *tetW* gene comprise at least one sequence selected from the group of SEQ ID NO:3 [GGA TAC TGA ACC] and SEQ ID NO: 6 [GTG GTT TAG TCT] is also an embodiment of the present invention.

The introduction of the "opal" and the "amber" stop codons into the protein coding region of the tetW gene represents two very different molecular events. In the case of the "opal" mutation a base was deleted whereas in the case of the amber a base was mutated (*in casu* from C to T pair, i.e. a base transition).

It should be emphasized that the tetW of a Bifidobacterium may be inactivated by other types of mutations in other sites of tetW. Insofar as *Bifidobacterium* strain contains a mutated, chromosomally encoded *tetW* rendering the strain sensitive to tetracyclines and which is obtainable by the method of the present invention the resulting tetracycline sensitive strains are embodiments of the present invention.

The most preferred embodiment of the present invention is the bacterial strain which is identified as *Bifidobacterium animalis* subspecies *lactis* strain CHCC8902 (Bb-12Tet-S139) and deposited on April 28, 2005 according to the Budapest Treaty on the International Recognition of the Deposit of Microorganisms for the Purposes of Patent Procedure with the Deutsche Sammlung von Mikroorganismen und Zellkulturen under accession number DSM17281. This tetracycline sensitive strain contains the "opal" mutation in its tetW. This strain is particularly preferred because it is a mutation of the well-known probiotic *Bifidobacterium* strains BB-12®. Furthermore CHCC8902 contains a single base deletion in tetW characterized of a relatively low reversion rate of less than 1.6 x 10⁻⁹ making it particularly suitable for ingestion in large numbers (see Example 4).

Another preferred embodiment of the present invention is the bacterial strain which is identified as *Bifidobacterium animalis* subspecies *lactis* strain CHCC9070 (DR10Tet-S9X) and deposited on April 28, 2005 according to the Budapest Treaty on the International Recognition of the Deposit of Microorganisms for the Purposes of Patent Procedure with the Deutsche Sammlung von Mikroorganismen und Zellkulturen under accession number DSM17282. This tetracycline sensitive strain contains the "amber" mutation in its tetW. This strain is also preferred because it is a mutation of the well-known probiotic *Bifidobacterium* strain DR10^{™} (CHCC7158). CHCC9070 (DR10Tet-S9X) contains a single base transition in *tetW*. As expected from the existing literature on base transitions the reversion rate is higher than e.g. deletion mutants. In the case of CHCC9070 which contains the back mutation rate 1.8 x 10⁻⁸. Although CHCC9070 is more prone to reversion than CHCC8902, the strain is still relatively stable and consequently suitable for ingestion.

*Bifidobacteria* and other lactic acid bacteria are commonly used as starter cultures serving a technological purpose in the production of various foods. The most well known industry using starter cultures is the dairy industry, thus in for the preparation of an ingestible material or a bacterial culture.

However as mentioned, *Bifidobacteria* can also serve as probiotics i.e. as non-pathogenic organisms, which have health benefits when taken orally in foods or capsules. Common targets of probiotic action are intestinal disorders (e.g. travelers diarrhea, antibiotic associated diarrhea) or intestinal symptoms (bloating, flatulence, discomfort). Yet, a wider range of benefits (e.g. anticholesterolemic, anticarcinogenic and immunostimulatory properties) is also discussed in the probiotic literature.

The terms "gastrointestinal tract" or "intestinal" are in the present context used interchangeably and relate to both the upper and lower gastrointestinal tract which includes the mouth, the esophagus, the stomach, the small intestines including the duodenum, the jejunum and the ileum, and the large intestines comprising colon and caecum.

The bacteria of this invention may be given in the form of a fermented food product or in a dosage forms formulated as a tablet (including chewable tablets), a capsule (of either the hard or soft type), a powder, a granulate, a liquid preparation, a suspension, a dried oral supplement, a wet oral supplement, a dry tube feeding formulation or wet tube feeding formulation.

In one embodiment the ingestible material is a fermented food or feed product prepared by use of the Bifidobacteria of the present invention. The fermented food or feed product may be further processed. In a number of situations it has been reported that bacteria produces health promoting compounds during fermentation. In such cases it might be advantageous to fractionate and or upconcentrate fractions of the fermented food product. One can even imagine that it in certain situations would be valuable to further process the fermented food product by pasteurization even though the beneficial Bifidobacteria are inactivated by such procedure.

However in general it is considered beneficial that the ingestible material comprises live *Bifidobacteria* in an amount from about 10⁵ cfu / g to about 10¹² cfu / g ingestible material, since living cells are a prerequisite for obtaining the probiotic effect.

It is contemplated that the *Bifidobacteria* of the present invention can be used for the preparation of a wide range of ingestible materials such as milk, curd, milk based fermented products, acidified milk, yoghurt, frozen yoghurt, milk powder, milk based powders, milk concentrate, cheese, cheese spreads, dressings beverages, ice-creams, ice-lollies or popsicles, fermented cereal based products, infant formulae and soybean milk.

A further important embodiment of the present invention is the use of the *Bifidobacteria* of the present invention to prepare a composition for the treatment, prevention of a disease, syndrome or condition, or for improving digestion of nutrients, or for improving the general health status of a human being or a vertebrate animal.

Since Bifidobacteria in general are considered as probiotic organisms (Yazid, 2000) the use of *Bifidobacteria* of the present invention as a probiotic is a preferred embodiment. The probiotic composition of the present invention can be any ingestible material selected from the group consisting of milk, curd, milk based fermented products, acidified milk, yoghurt, frozen yoghurt, milk powder, milk based powders, milk concentrate, cheese, cheese spreads, dressings beverages, ice-creams, fermented cereal based products, infant formulae, tablets, liquid bacterial suspensions, dried oral supplement, wet oral supplement, dry tube feeding or wet tube feeding that is produced by use of the *Bifidobacteria* of this invention.

In a further embodiment, the composition further comprises a pharmaceutically acceptable carrier. As used herein, the term "pharmaceutically acceptable carrier" means one or more solid or liquid filler diluents or encapsulating substances which are suitable for administration to a human or an animal and which is/are compatible witch the probiotically active organisms. The term "compatible" relates to components of the pharmaceutical composition which are capable of being commingled with the probiotic in a manner enabling no interaction because it would substantially reduce the probiotic efficacy of the organisms selected for the invention under ordinary use conditions. Pharmaceutically acceptable carriers must be of a sufficiently high purity and a sufficiently low toxicity to render them suitable for administration to humans and animals being treated.

In useful embodiments, the ingestible material according to the invention is suitable for preventing or treating a disease, syndrome or condition is selected from the group consisting of antibiotic-associated disorders, gastroenteritis, diarrhea including traveller's diarrhea and acute infantile diarrhea, lactose intolerance, gastrointestinal infections and colonization of the gastrointestinal tract by pathogenic bacteria including Helicobacter pylori and Clostridium difficile, irritable bowel syndrome (IBS), inflammatory bowel disease (IBD) and other immunomodulative syndromes, colonic cancer, urogenital infections and tumors, vaginal infections, allergy (especially atopic eczema), vaccination, cholesterolemia and hypertension.

In further useful embodiments, the ingestible material according to the invention is suitable for preventing or treating infections with pathogens such as e.g. *Heliobacter pylori, Campylobacter pyloridis, Staphylococcus aureus, Staphylococcus epidermidis, Strepto*coccus pyogenes, *Streptococcus pneumoniae,* Enterococcus *faecalis, Hemophilus influenzae, Escherichia coli, Klebsiella pneumoniae, Enterobacter* cloacae, *Citrobacter freundii, Serratia marcescens, Pseudomonas aeruginosa and Pseudomonas maltophilia, Salmonella sp.* and fungi such as *Candida albicans* and *Aspergillus fumigatus,* and combinations of these species.

In recent years rotaviruses and other enteric viruses have been identified as a major cause of infectious diarrhea. Interestingly both *Bifidobacterium* BB-12® and HN019 (DR10^{™}) have been shown effectively to prevent or treat infections also with these pathogens. Thus in useful embodiments, the ingestible material according to the invention is used for preventing or treating infections with rotaviruses and other enteric viruses.

It may be useful to combine two or more of the above assumingly probiotically active organisms, such as e.g. a preparation comprising a Lactobacillus species and a *Bifidobacterium* species.

### Performance of the mutant strains and their benefits to man.

The *Bifidobacterium animalis* subsp. *lactis* BB-12® (CHCC5445), is an extremely important strain for the health and well being of mammals due to its probiotic capabilities (e.g. immune stabilizing effect in humans, controlling of a balanced microflora in the digestive tract thereby reducing or acting as inhibitors of various epidemiologic syndromes, etc.). Also the *Bifidobacterium animalis* subsp. *lactis* HN019 (DR10^{™}, CHCC7158), has an impressive record of probiotic activity. Although both of these strains harbor an active gene encoding resistance to tetracycline, the most prominent bacterial antibiotic resistance found in nature (Chopra and Roberts, 2001), both have for many years been used in food production, and to our knowledge without causing any harm on the contrary only positive effects have been ascribed to the use of these strains. However, the fact that both strains contain an active *tetW* in their genome does possess the theoretical possibility of transferring the tetracycline resistance to other - and perhaps harmful bacteria in the human digestive system. The risk of this increase if ingested donor bacteria survive in the gut in large numbers, as is the case with the typical use of probiotic bacteria. Inactivation of the *tetW* gene in the two variants, as it has been demonstrated here, eliminates the risk of a horizontal transfer of functional antibiotic resistant genes. Apart from the lesion in the *tetW* the two tetracycline sensitive strains are probably isogenic with their mother strains as suggested under Example 5 and Example 6, thereby it can be assumed that the two tetracycline sensitive strains possess most if not all the features that make BB-12® and HN019 (DR10^{™}) probiotic.

Performance and application properties are ongoing in regards to production, stability including animal experiments (Example 7) to confirm the probiotic characteristics of the Bb12Tet-S139 (CHCC8902) and the DR10Tet-S9X (CHCC9070).

In addition, laboratory tests involving two-dimensional gel-electrophoresis for further characterization of the mutant strains are being performed to verify the isogenic background with the wild type strains.

Conclusion
1) - inactivation of the tetracycline resistance gene, *tetW,* from *Bifidibacterium animalis* ssp *lactis* BB-12® and Bifidibacterium *animalis* ssp *lactis* HN019 (DR10™) was established by a combination of the intercalating mutagen, ethidium bromide, and successive ultra violet irradiation.
2) - two of the resulting tetracycline sensitive isolates one originating from BB-12 ® one originating from HN019 (DR10^{™}) were incapable of growing in MRS broth with tetracycline in a concentration above 0.5 µg/ml.
3) - characterization of one of the mutant isolates, Bb12Tet-139 (a derivative of CHCC5445) demonstrated a frame shift at nucleotide position # 1405 in the *tetW gene* immediately resulting in an opal stop codon 170 amino acids short of the gene product. In the other mutant, DR10Tet-S9X (a derivative of CHCC7158) an amber stop codon was introduced (nucleotide position # 424), 498 amino acids short of the gene product.
4) - the reversion rate for Bb12Tet-S139 is less than 1.6 x 10⁻⁹ and the back mutation rate for Bb12Tet-S9X is 1.8 x 10.⁻³.
5) - growth, acidification rates DNA-profile and the transcriptome (Example 6) of the *tetW* mutants were not different to the respective wild types strains.
6) - analyses of the mutant isolates did not disclose any re-arrangements or modifications, but for *tetW,* of the genomic DNA as judged from DNA fingerprinting- and transcriptomics analyses.

The invention is further illustrated in the following examples and tables wherein

Table 1. Is a description of oligonucleotides used for PCR analyses and DNA sequencing of the tetracycline resistance-encoding *tetW gene* of BB-12® and HN019.

Table 2. Is the tetW DNA sequence and the upstream transposase gene, *tps,* from *Bifido-bacterium animalis* subsp. *lactis* BB-12® (SEQ ID 22).
Nucleotide sequence of the inactive *TetW* transposon from *Bifidobacterium animalis subsp. lactis* BB-12®. The sequence was obtained by sequencing at Chr. Hansen A/S and verified by comparison to a similar obtained from from Danone
Upstream is the transposase encoding open reading frame, nt. # 4 -966, with the amino acid depicted under the DNA sequence (M. W. approx. 35 kDa). The nt. sequence # 1318-2334 is the tetracycline resistant encoding gene, *tetW*, with the amino acid outlined under the DNA sequence. (M. W. approx. 70 kDa).
The amber mutation at nucleotide (nt) position # 1741 (nt # 424 relative to the start codon of *tetW)* for the tetracycline sensitive strain, DR10Tet-S9X (CHCC9070), is indicated above the DNA sequence.
The frameshift mutation at nt position # 2722 (nt #1405 relative to the start codon) for the tetracycline sensitive strain, Bb12Tet-S139 (CHCC8902), is indicated above the DNA sequence.
*): indicates a stop codon.

### EXAMPLES

### Example 1: Inactivation of the tetW gene in two probiotic strains of Bifidobacterium animalis subspecies lactis expressing resistance to tetracycline.

A strong and dual mutagenic approach was used to inactivate the intrinsic tetracycline resistance of the genome of *Bifidobacerium animalis* ssp. *lactis* strain BB-12® and strain HN019 (DR10^{™}). This included treatment of the cells with the mutagen, ethidium bromide (EtBr) followed by ultra violet irradiation in a more forceful scale than normally when the mutagens are used individually.

### Strains and culture conditions.

Strains of *Bifidobacterium animalis* subspecies *lactis* BB-12® and HN019 (DR10^{™}) were obtained from the culture collection of Chr. Hansen A/S,Hørsholm, Denmark. *B*. *animalis* subsp. *lactis* strain BB-12® has the accession number CHCC5445 in the Hansen culture collection, it is deposited with the Deutsche Sammlung von Mikroorganismen und Zellkulturen (DSMZ) under accession number DSM15954. *B. animalis* subsp. *lactis* strain HN019 (DR10^{™}) was isolated from a commercial available infant formula product labeled Fernleaf DR-10 bifidus and sold in Taiwan during 2000. It has the accession number CHCC7158 in the Hansen culture collection and is deposited with DSMZ under accession number DSM17280.

The two strains which are resistant to tetracycline (at least 15 µg/ml, as determined by the Etest procedure, Danielsen and Wind, 2003), were grown routinely under anaerobically conditions at 37°C in Difco-MRS broth (deMan 1960), supplied with 0.05% Cysteine hydrochloride (Cysteine-HCl, Merck chemicals) as well as on MRS agar (1.5% agar) with the same concentration of Cysteine-HCl and 15 µg of tetracycline per ml.

### Mutagenic treatment of Bb-12

An aliquot (2%) of an overnight (on) grown BB-12® in MRS was transferred to fresh MRS broth (10 ml without tetracycline) containing 20µg/ml ethidium bromide (EtBr) and incubated to optical density [OD] at 600 nm, 0.35. The exponentially growing cells were then subjected to UV-irradiation (UV cross-linker, Stratagene) in an open Petri dish for 5 min (70 mJ/cm²). The UV treatment was repeated once after a dark interval of 5 min at room temperature. The treatment was adjusted to obtain a lethality of approximately 90%. The lethality was assessed as follows. The viability of the cells immediately after the EtBr-UV treatments was determined by cell plating on MRS agar without tetracycline and the lethality was calculated from the observed CFU.

One ml of the mutagenised culture was then transferred to 9 ml fresh MRS without the addition of EtBr and were then allowed to grow for a period of 16 hours at 37°C in complete darkness, at which point aliquots of the treated cells were re-inoculated (1% [vol/vol]) into fresh MRS broth and allowed to grow for an additional 16 hours.

### Example 2: Selection of tetracycline-sensitive variants of two probiotic strains of Bifidobacterium animalis subspecies lactis.

### Screening procedure

Screening for tetracycline sensitive isolates was performed after an ampicillin enrichment procedure (Miller, 1972) adapted to *Bifidobacteriaceae* was performed. Briefly, the ampicillin enrichment procedure was performed by transferring an aliquot (1 %) of an overnight culture to fresh MRS medium (10 ml) and growing the cells to an OD₆₀₀ of 0.2 without the addition of tetracycline. 0.5 ml of this culture was used to inoculate 10 ml of MRS broth containing 10 microgram tetracycline/ml and incubated for 2 hrs at 37°C at reduced oxygen tension, after which ampicillin was added to a final concentration of 150 microgram/ml, and the culture was continuously incubated for 16 hrs at 37°C. As ampicillin kills only growing cells (i. e., Tet^{R} cells) and not nondividing cells (i. e. mutant Tet^{S} cells), the addition of ampicillin can be considered an enrichment step for the subsequent isolation of Tet^{S} mutants. Cells were harvested by centrifugation (4,000 x G for 5 min at 4°C) and washed twice with fresh MRS broth.

Following the ampicillin enrichment the screening for tetracycline sensitive isolates was performed by plating aliquots of the washed cells on MRS agar in an appropriate dilution to give approximately 150 colonies per plate after incubation at 37°C for 20 hours.

Tetracycline sensitive colonies were identified by replica plating on MRS agar without antibiotics and MRS agar containing 10 µg of tetracycline per ml, on which the tetracycline sensitive isolates were unable to grow. Finally, the tetracycline sensitive colonies were cultured in MRS broth. Total genomic DNA was isolated from both the tetracycline sensitive clones and the tetracycline resistant strain for intensive characterization.

The replica screening resulted in 155 tetracycline sensitive isolates from CHCC5445 of a total of approximately 4,000 screened colonies. From CHCC7158, 43 tetracycline sensitive colonies were isolated out of approximately 1,000 cells. All these colonies were further tested in liquid MRS broth containing tetracycline (10 µg/ml). A substantial fraction of false positives (approx. 85 %), managed to grow under these conditions. The remaining tetracycline sensitive isolates were then subjected to Etest susceptibility screening according to the methods described by M. Danielsen and A. Wind (2003), to determine their tetracycline sensitive threshold. In most cases it ranged between 2 and 4 µg/ml. Only two isolates, one derived from CHCC5445, named Bb12Tet-S139, and one derived from CHCC7158, named DR10Tet-S9X, demonstrated a higher sensitivity to tetracycline with an MIC value of 0.5 µg/ml.

### Example 3: Molecular characterization of the tetracycline-sensitive derivatives of two probiotic strains of Bifidobacterium animalis subspecies lactis.

### PCR amplification and DNA sequencing of the tetW gene

Genomic DNA was prepared from wild type BB-12® and the two tetracycline-sensitive isolates by the use of the Easy-DNA protocol for isolation of DNA from gram-positive bacteria according to the manufacturer's (Qiagen) instructions.

The *tetW* gene of the sensitive variants was characterized by PCR analyses according to the protocol of Innis and Gelfand (1990), and DNA sequencing to test for possible mutations in that gene. The entire open reading frame (ORF, approx. 2.0 kb) of *tetW* was amplified from each isolate in three overlapping fragments (A, B and C) of approx. 75 bp each with three sets of primers (table 1, table 2).

Fragment A (approx. 785 bp) was amplified with sense primer: *tetWx*.D1, derived from a sequence 296 bp upstream of the start codon of the *tetW* gene and antisense primer: *tetWx*.R2, derived from the ORF of *tetW.* Fragment B (approx. 935 bp) was amplified with sense primer: *tetWx*.D4 and antisense primer: *tetWx*.R5 of the ORF of *tetW*. Fragment C (920 bp) was amplified with sense primer: *TetWx*.D3 derived from the ORF and antisense primer: tetWx.R4 262 bp downstream of the termination codon of the *tetW* gene.
The amplified fragments from the three PCR reactions were subjected to agarose gel-electrophoresis (0.7%) and staining in EtBr and identified with UV illumination. The bands corresponding to the three amplified gene fragments were excised from the gel and DNA extracted (QlAquick gel extraction kit from Qiagen). The purified PCR products were cloned into the plasmid vector, pCR2.1-TOPO (Invitrogen), for nucleotide sequence determination using the M13 forward and reverse primers.
DNA sequencing of the *tetW* genes from each of the individual tetracycline sensitive isolates with Etest-values of 2-4 µg/ml demonstrated that the tetracycline resistance gene was not affected or mutated in these isolates, and was 100% homologous to the *tetW* gene in the wild type BB-12® depicted in Table 2.

Sequencing of the *tetW* genes from the two isolates with tetracycline Etest-values of 0.5 µg tetracycline/ml showed that the *tetW gene* was affected in both cases. The Bb12Tet-S139 (a derivative of CHCC5445) demonstrated a frameshift at nucleotide position #1405, where a thymidine residue was deleted as illustrated below and in table 2.

| | |
|---|---|
| Amino acid sequence: | - Ser Leu Gly Tyr Leu Asn Gln Ser |
| BB-12® (CHCC5445) nt # 1393: | - TCG CTG GGA TAC TTG AAC CAG AGT- |
| | |
| Bb12Tet-S139 (CHCC8902) nt # 1393: | - TCG CTG GGA TAC TGA ACC AGA GTT - |
| Amino acid sequence: | - Ser Leu Gly Tyr opal (stop codon). |

The illustration above shows a partial sequence of the *tetW* gene in CHCC5445. The underlined thymidine residue in BB-12® (nt: 1405 in the *tetW* sequence) is deleted in the frameshift mutant, Bb12Tet-S139. resulting in an opal stop/nonsense codon 170 amino acids short of the wild type *tetW* gene product.
[nt #1405 has the nt position number # 2722 in the sequence in table 2, SEQ ID XX].

DNA sequencing of the *tetW* gene from the other tetracycline sensitive isolate DR10Tet-S9X, a derivative of strain HN019 (CHCC7158), demonstrated a transition of a cytidine to a thymidine residue at nucleotide position # 424 thus immediately generating an amber translational stop codon as depicted below and in Tabel 2.

| | |
|---|---|
| Amino acid sequence: | - Gln Ser Val Val Gln Ser Val Arg - |
| DR10™ (HN019, CHCC7158) nt # 412: | -CAG AGC GTG GTT CAG TCT GTT CGG- |
| | |
| DR10Tet-S9X (CHCC9070) nt # 412: | -CAG AGC GTG GTT TAG TCT GTT CGG- |
| Amino acid sequence: | - Gln Ser Val Val amber (stop codon). |

The illustration above shows a partial sequence of the *tetW* gene in CHCC7158, which is identical to the *tetW* gene in CHCC5445. The underlined cytidine residue in HN019 (nt: 424) is substituted with a thymidine residue in the mutant strain, resulting in an amber stop/nonsense codon 498 amino acids short of the wild type *tetW* gene product. [nt # 424 has the nt position number # 1741 in DNA sequence in in table 2, SEQ ID XX].

### Example 4: Genetic stability of the tetracycline sensitive isolate Bb12Tet-S139 (CHCC8902) and DR10Tet-S9X(CHCC9070)

### Determination of back mutation rates by cell plating on MRS agar with tetracycline

One ml of an over night grown culture of the Bb12Tet-S139 mutant strain (1.6 x 10⁻⁹ cells/ml) was spread with 50 µl each onto 20 Petri dishes (diameter: 13.8 cm) with MRS agar supplied with tetracycline (15 µg/ml). After 24 hours anaerobic incubation at 37°C no colonies could be detected on the plates demonstrating that the reversion rate is less than 1.6x10⁻⁹.

The same stability testing was performed for the DR10Tet-S9X strain. An over night culture of this strain (1.1 x 10⁻⁹ cells/ml) was likewise spread with 50 µl each onto 20 Petri dishes with MRS agar supplied with tetracycline (15 µg/ml). After the incubation period 19 tetracycline resistant colonies were detected. The reversion rate for the amber mutation was calculated to 1.8 x 10⁻⁸.

### Example 5: Physiological and phenotypic testings of the two tetracycline sensitive strains, Bb12Tet-S139 (CHCC8902) and DR10Tet-S9X(CHCC9070).

### Growth conditions of the mutant strains in MRS broth.

Growth of the Bb12Tet-S139 and DR10Tet-S9X were compared (in triplicates) to their mother strains, CHCC5445 and CHCC7158, respectively, grown under similar conditions in MRS broth (200 ml with Cysteine-HCl) over a period of 24 hours. Samples for measuring the optical density at 600 nm were monitored all along the incubation period. The growth rates for both mutants were similar to those of the parent wild type strains indicating that the mutagenic treatment of the tetracycline sensitive isolates did not seem to hamper genes in their fermentative pathways.

### Acidification rates of the two tetW mutants.

The acidification (MRS broth), that is the conversion of pyruvate to lactic acid, was monitored (in duplicates) over a period of 6 hours and compared to the respective mother strains grown under the same conditions. No obvious difference in the rate of acidification was observed between the mutant derivatives of CHCC5445 and CHCC7158 and their mother strains.

### DNA-fingerprinting analysis.

Pulsed-field gel-electrophoresis of the genomic Spel- and Xbal-digested DNA from the two mutant *tetW* strains, was performed according to standard methods (Hung and Bandziulis, 1990), and did not reveal any rearrangement of the Spel- or *Xba*I-digested chromosomal pattern when compared to the respective wild type strains. This adds evidence for an overall isogenic background of the mutants and the mother strains.

### Example 6: Genome wide gene expression (transcriptomic) analysis shows that the gene-expression of Bb12Tet-S139 is indistinguishable from BB-12®.

### Materials & Methods

### Design and production of whole genome microarrays for Bb12

We have previously sequenced and set up whole genome microarrays for BB-12® (Garrigues et al., submitted). Briefly, BB-12® was shotgun sequenced, resulting in 56 contigs. Through further assembly analysis it was clear that only a couple of percent of sequence was missing. Within the almost 2.0 Mb genome sequence 1612 putative CDSs (coding sequences) were identified. A specific 65-75mer oligo was designed for each gene with a few exceptions, such as very small putative genes. For a couple of genes several oligos were designed. In all, 1569 oligos were designed from the sequence. In addition more than 100 control oligos were designed. Oligos were printed on UltraGAPS slides (Corning B. V., Schiphol-Rijk, The Netherlands) in four replicate copies as described previously (Pedersen et al. 2005).

RNA samples were collected in RNAprotect (QIAGEN, Valencia, CA, USA) to stabilize the expression profile and total RNA was isolated (RNeasy, QIAGEN). 10 µg of RNA was copied into cDNA using the CyScribe Post-Labelling Kit with 1.65 µg of random nonamer as primer (Amersham Biosciences, Hillerød, Denmark). The test condition was labeled with Cy5 and the reference condition with Cy3. The two samples were pooled and half of this was hybridized to the array. Instead of the 50% formamide recommended in the protocol 60% was used (these conditions were established previously using the control oligos). After around 18h of hybridization the array was washed (Corning B.V.), scanned, and pre-analyzed as described previously (Pedersen et al. 2005). The array data was imported into Acuity 4.0 (Axon Instruments Inc., Union City, CA, USA) where it was LOWESS normalized. A dataset was created with all identified spots. Oligos where only 0, 1, or 2 of the 4 replicate spots had been identified were removed. Similarly, oligos where the standard deviation between the normalized log2(ratio)s was >0.5 were removed.

### Results

The gene expression of Bb12Tet-139S was compared to that of Bb12 using the whole genome microarrays. Stationary cultures of both strains were inoculated at 1% (OD600=0.06) into fresh MRS+0.05% cysteine · HCl preheated at 37°C. OD600 was then measured every 30min. In the OD range of 0.1-1.0AU (absorbing units) growth was exponential. The specific growth rate, p, was 0.50h⁻¹ (R2=0.9977) and 0.51h⁻¹ (R2=0.9953) for BB-12® and Bb12Tet-S139, respectively. Hence, there is no significant difference between the growth rates of the two strains. From RNA samples at OD=1.0AU (after six and half hours of growth) microarrays were produced. Bb12Tet-S139 and BB-12® were the test and reference condition, respectively. Of the 1569 genes represented on the microarray significant regulatory data was obtained from 1271 genes.

As a rule of thumb a gene is often considered to be differentially expressed if it is >2-fold up or down-regulated between two conditions (see for example Pedersen et al. 2005). None of the 1271 genes in the dataset were more than >2-fold differentially expressed. In comparison, when Bb12 was exposed to 0.1% bile salt 86 and 123 genes were >2-fold up and down-regulated, respectively (Garrigues et al., submitted). Among these genes, 17 and 27 were even >4-fold up and down-regulated, respectively. This shows that if cell metabolism is perturbed dramatic changes may occur with regards to gene expression. Similarly, when 1% of fructooligosaccharide (FOS) was added to the medium, only 2 genes were >2-fold differentially expressed. These 2 genes encode proteins involved in FOS utilization and were 5-fold up-regulated (Garrigues et al., submitted). This latter experiment shows that the growth conditions can be reproduced and that perturbations affecting particular parts of metabolism can be identified. By combining the above results it is clear that with regards to gene expression and general physiological state no adverse effects are observed in Bb12Tet-S139 compared to BB-12®. Based on this it can be assumed that Bb12Tet-S139 behaves as BB-12® except for the sensitivity to tetracycline.

### Example 7: Probiotic activities - Prevention/reduction of Salmonella infection in mice.

### Background:

It has previously been shown in mice that *Bifidobacterium* BB-12® (CHCC5445) has antagonistic effects against *Salmonella enteritidis* subsp. *typhimurium* and thereby has been shown to reduce the pathological consequences for the mouse (significantly higher survival in probiotic groups compared to placebo). The intention is to repeat the studies with both *Bifidobacterium* BB-12® (CHCC5445) and Bb-12Tet-S139 (CHCC8902) to show that CHCC8902 is probiotic and shows antagonistic effects against Salmonella enteritidis subsp. typhimurium to the same level as CHCC5445.

### Challenge study in mice:

21 days old conventional mice are divided into 3 experimental groups of each 10 mice. The mice will be treated daily by gavage with 0.1 mL reconstituted skim milk containing 1 billion CFU of Bb-12Tet-S139 (CHCC 8902), BB-12® (CHCC 5445) or placebo for 5 days. At day 5 the mice are challenged with a single dose of 100 CFU *Salmonella enteritidis* subsp. *typhimurium* (grown in liquid brain heart infusion (BHI) medium (Difco) at 37°C). Mice will be inoculated by the orogastric route with 0.1 ml of a bacterial suspension containing about 100 viable cells. The probiotic treatment is continued for 28 days with registration of well being, clinical symptoms and deaths every day and pathological examination is performed on dead animals. At the end of the experiments all remaining mice will be sacrificed by ether inhalation and macroscopical pathology will be performed. Feces will be collected from all animals before the trial and at day 1, 7, 14 and 28 of the trial. Probiotic bacteria will be recovered from feces using selective plating followed by specific identification of colonies using a PCR technique.

### Evaluation.

Differences in survival/disease/death and pathological changes between the groups will be analyzed to show a protective ability of the probiotic bacteria towards the Salmonella infection. Statistical evaluation will be performed at the level of significance set at P<0.05.

### REFERENCES

Barbosa, T. M., K. P. Scott, and H. J. Flint. 1999. Evidence for recent intergeneric trans-fer of a new tetracycline resistance gene, tet(W), isolated from Butyrivibrio fibrisolvens, and the occurrence of tet(O) in, ruminal bacteria. Environ. Microbiol. 1:53-64.
Billington, S. J, J. G. Songer, and B. H. Jost (2002) Widespread Distribution of a Tet W Determinant among Tetracycline-Resistant Isolates of the Animal Pathogen Arcanobacterium pyogenes. ANTIMICROBIAL AGENTS AND CHEMOTHERAPY, 46: 1281-1287.
Cai et al (2000) Microbial ImmunoL 44, 815-820
Chopra, I., and M. Roberts (2001). Tetracycline antibiotics: mode of action applications, molecular biology, and epidemiology of bacterial resistance". Microbiol. Mol. Biol. Rev. 65:232-260.
Chopra, I., and M. Roberts. 2001. Tetracycline antibiotics: mode of action, applications, molecular biology, and epidemiology of bacterial resistance. Microbiol. Mol. Biol. Rev. 65:232-260.
de Man, J. C., M. Rogosa, and M. E. Sharpe. 1960. A medium for the cultivation of lactobacilli. J. Appl. Bacteriol. 23:130-135.
Garrigues, C., Stuer-Lauridsen, B., and E. Johansen, submitted to Australian Journal of Dairy Technology.
Hung, L.. and R. Bandziulis, Promega Notes 24: 1-2, 1990, Promega, Madison, Wis. Lachman, L. et al (Ed.) 1986. The Theory and Practice of Industrial Pharmacy. Third Edition. Lea & Febiger, Philadelphia.
List no. 62 (1997) Int. J. Syst. Bacteriol 47, 915-916
M. A. Innis and D. H. Gelfand (1990) Optimization of PCRs p. 3-12. In M. A. Innis, D. H. Gelfand, J. J. Sninsky, and T. J. White (ed), PCR protocols, a guide to methods and applications. Academic Press, San Diego, Calif.
M. Danielsen and A. Wind. 2003 "Susceptibility of Lactobacillus spp. to antimicrobial agents". Int. J. Food Microbiol. 82:1-11.
Masco et al (2004) Int. J. Syst. EvoL MicrobiaL 54, 1137-1143
Meile et al (1997) System. AppL MicrobioL 20, 57-64
Miller, J. H. (1972) "Experiments in molecular genetics", p 230-234. Cold Spring Harbor Laboratory, Cold Spring Harbor, N. Y.
Minutes (2001) Int. J. Syst. EvoL MicrobioL 51, 259-261
Moubareck, C. et al. (2005) Antimicorbial susceptibility of Bifidobacteria. J. Antimicrobial Chemotherapy 55: 38-44.
Pedersen, M. B., Iversen, S. L., Sørensen, K. I., and E. Johansen. 2005. The long and winding road from the research laboratory to industrial applications of lactic acid bacteria. FEMS Microbiology Reviews, accepted.
Scott, K.P., C. M. Melville, T. M. Barbosa, and H. J. Flint (2000) Occurrence of the New Tetracycline Resistance Gene tet(W) in Bacteria from the Human Gut. ANTIMICROBIAL AGENTS AND CHEMOTHERAPY, 44: 775-777.
Silliker, J. H. et al (Ed.) 1980. pH and acidity. IN: Microbial Ecology of Foods, vol. 1 pp. 92-111. Academic Press, New York.
WO 97/16198, (Chr. Hansen A/S Biosystems), 9 May 1997
Yazid, A.M., A.M. Ali, M. Shuhaimi, V. Kalaivaani, M.Y. Rokiah and A. Reezal (2000) An-timicrobial susceptibility of bifidobacteria. Letters in Applied Microbiology 2000, 31, 57-62
Zhou, J.S., C.J. PillidgeC, P.K. Gopal and H.S. Gill (2005) Antibiotic susceptibility profiles of new probiotic Lactobacillus and Bifidobacterium strains. International Journal of Food Microbiology 98:211-217.
Regarding Deposited Microbial Organisms [EXPERT SOLUTION]
For all deposited microbial organisms mentioned in the present patent application the following applies.

In respect to those designations in which a European Patent is sought a sample of the deposited microorganism will be made available until the publication of the mention of the grant of the European patent or until the date on which application has been refused or withdrawn or is deemed to be withdrawn, only by the issue of such a sample to an expert nominated by the person requesting the sample, and approved either i) by the Applicant and/or ii) by the European Patent Office, whichever applies. (Rule 28 (4) and 28 (5) EPC).

**Table 1**

| | | |
|---|---|---|
| Oligonucleotides used for PCR analyses and DNA sequencing of the tetracycline resistance-encoding *tetW* gene of Bb-12. | | |

| **Primer** | **Sequence (5' to 3')** | **Nucleotide positions*** |
|---|---|---|
| Stps.D5 | GAGCATCGCTTAATTCCTTCGAGGGG | -394 : -369 |
| StetW.D | CGAGGGGACTTTGGCCCACCGCTGGGTGG | -375 : -347 |
| StetW.D1 | GCAGGCAGGCACGTCCATCCGCCTC | -199 : -175 |
| Stps.R3 | GGCTTCTGCCACCGTGGCCTCGTCAC | 234 : 209 |
| tpsW.R1 | CCTGTCCTTGTCCACGCCGATATGCGC | 554 : 526 |
| tetWx.D1 | CGGCACCTGCTGTATAATGCGGATTGTGGC | 1022 : 1051 |
| tetW.Dup | GTGGGAACAAAGGATTATGATAGTCCC | 1092 : 1118 |
| tetWx.D4 | GGCTGGCGTTGATTTGCAGAGCGTGG | 1713 : 1738 |
| tetW.D500 | GCAGACGGTGTCGCTGTCCCCGG | 1782 : 1804 |
| tetWx.R2 | CCGGGGACAGCGACACCGTCTGC | 1804 : 1782 |
| tetWx.D3 | GGAGCGGCCGCTCAAAGCAGCCAGCC | 2580 : 2605 |
| betWx.R3 | GGCTGGCTGCTTTGAGCGGCCGCTCC | 2605 : 2580 |
| tetWx.R5 | GCCCAAAACGGGTTGGGCGGCACCTCG | 2645 : 2619 |
| tetW.R500 | CCAGCCCGTAACGGATACCATCCC | 2773 : 2750 |
| tetWx.R4 | GATGGTCGCATGATCGGCGGGGTCACTCCC | +262 : +233 |

| | | |
|---|---|---|
| *Numbering is based on the DNA sequence illustrated in table 2. The prefix "D" indicates a direct primer derived from the sense strand. The prefix "R" indicates a reverse prime complementary to the sense strand. | | |

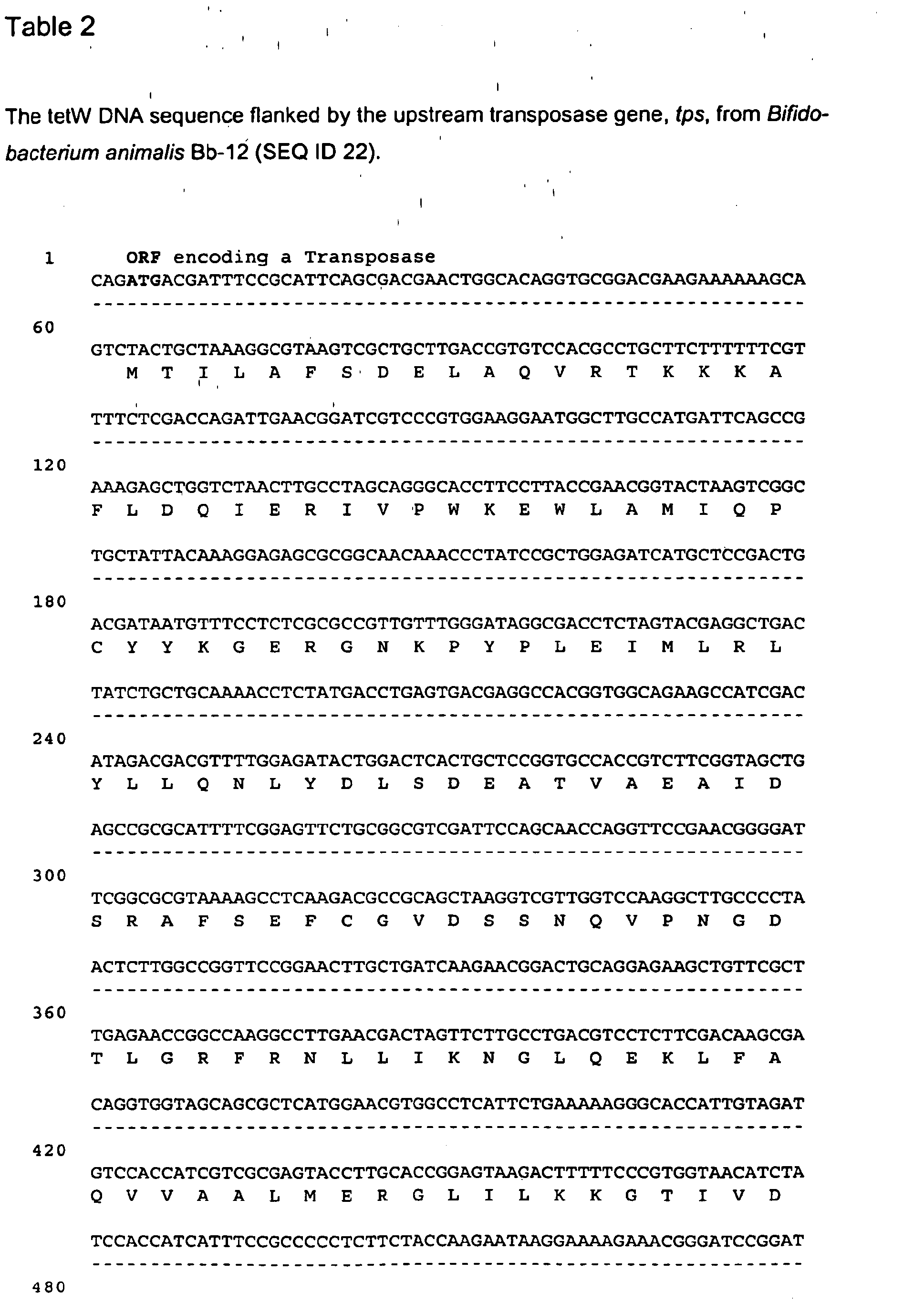

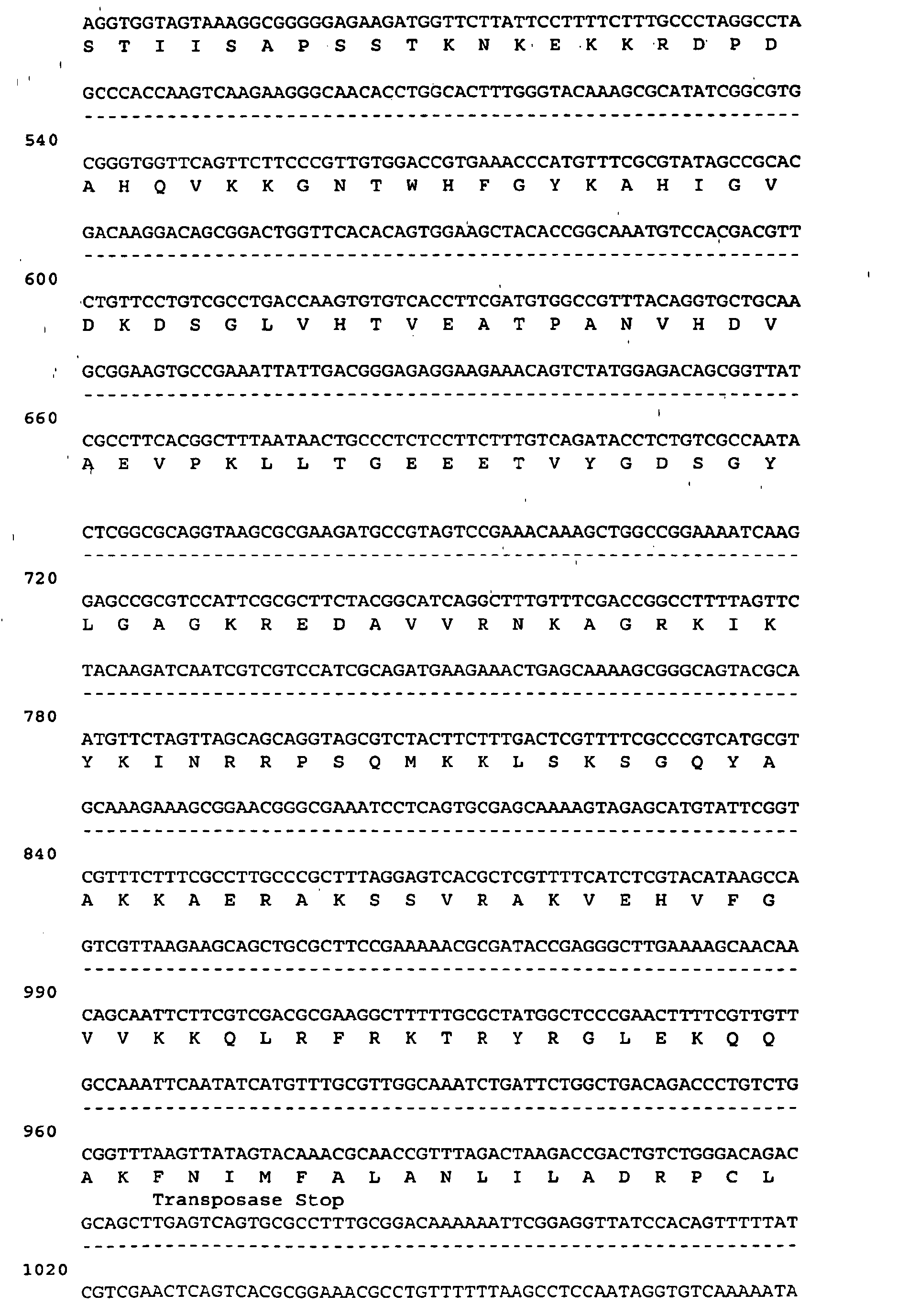

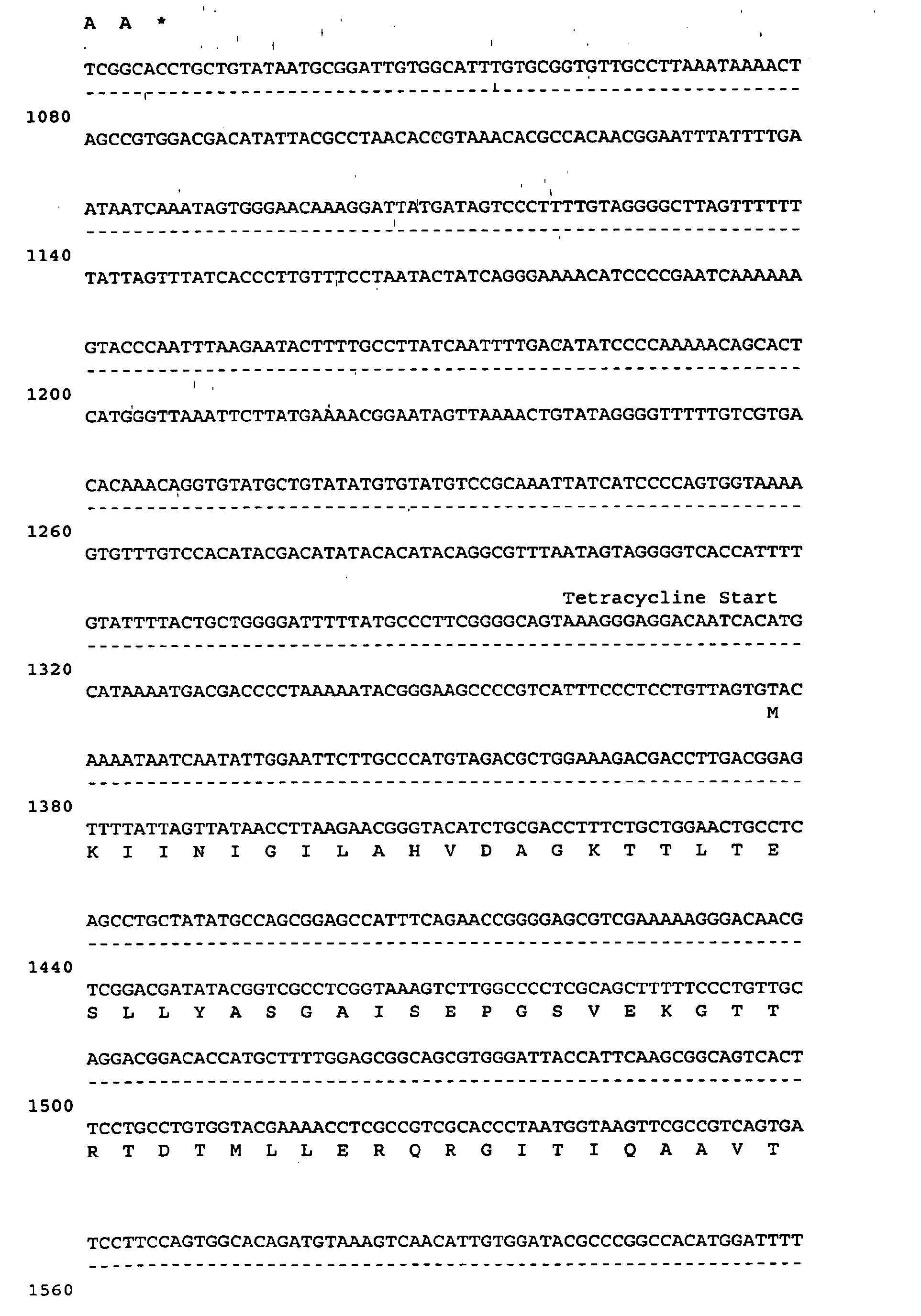

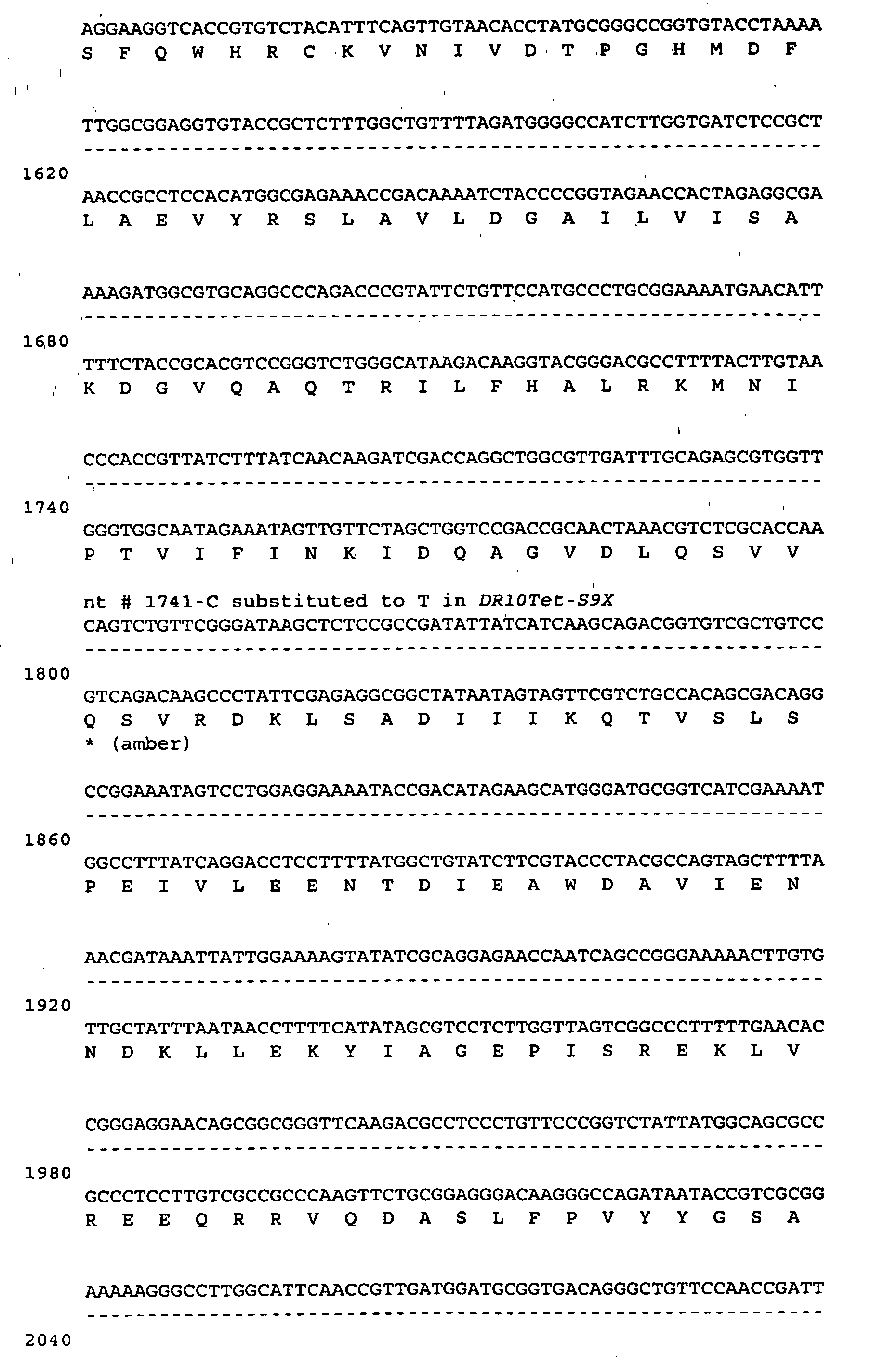

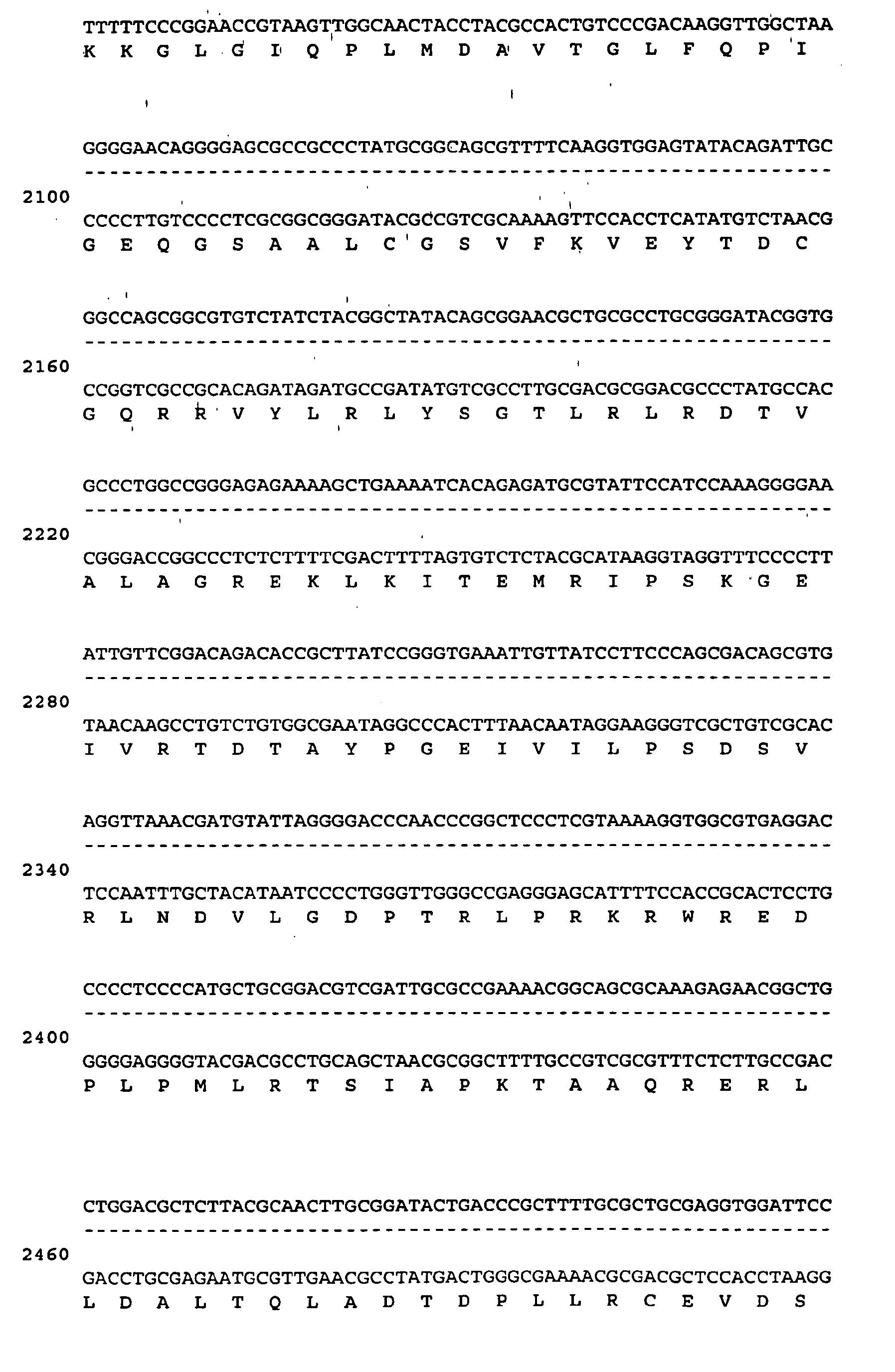

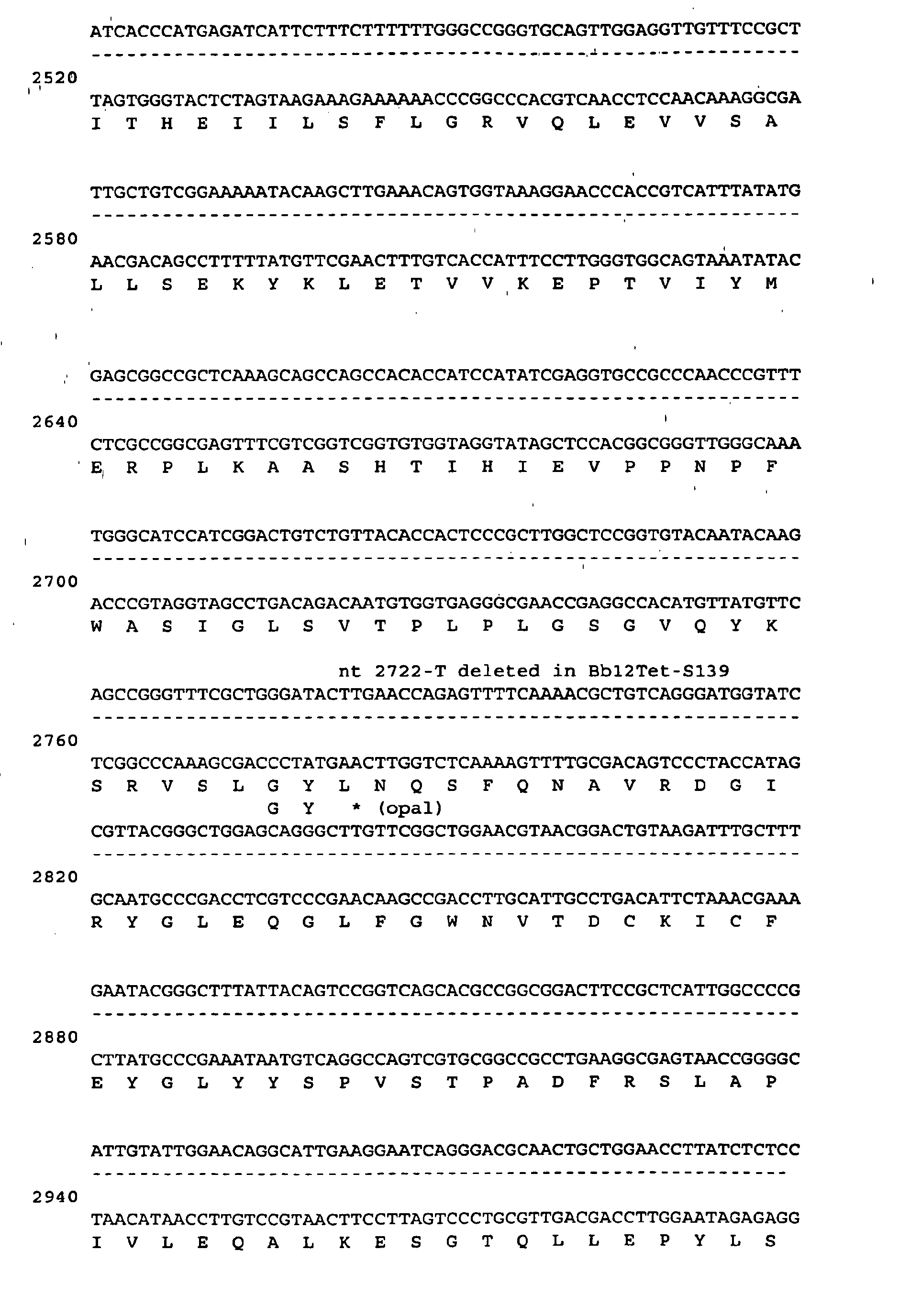

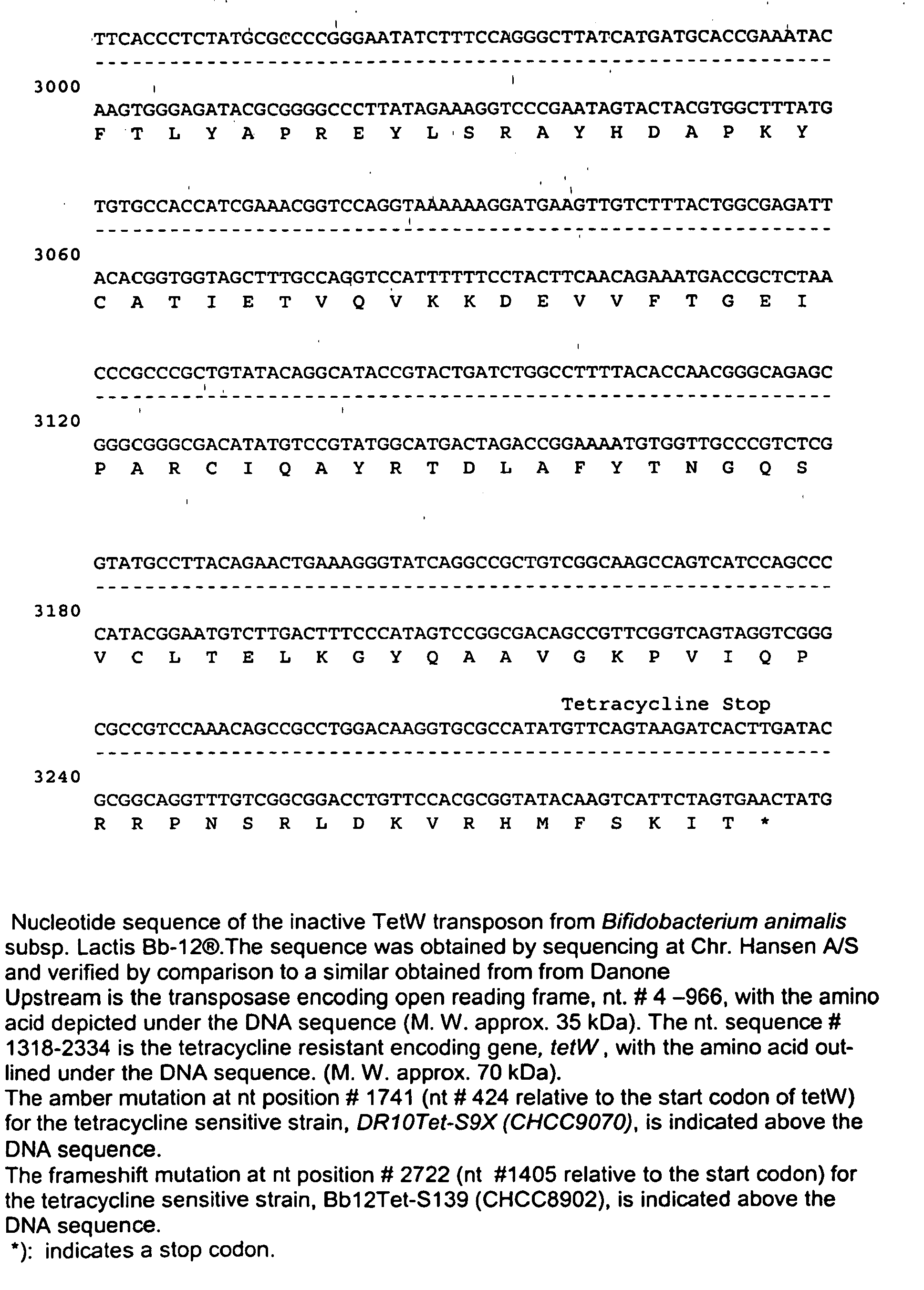

## Claims

1. A method of isolating a strain of *Bifidobacterium* sp. containing a mutated *tetW* on its chromosome, said mutation renders the strain sensitive to tetracyclines and said strain is isolated from a tetracycline-resistant bacterial progenitor strain wherein the antibiotic resistant phenotype is caused by the expression of *tetW* stably integrated in its chromosome, said method comprising subjecting the cells to a chemical mutagen and a physical mutagen, and
wherein the method comprises the steps of:
1) culture the progenitor cells to obtain a culture of exponential growing cells,
2) transfer an aliquot of the cells to fresh medium containing ethidium bromide (EtBr),
3) transfer the culture to one ore more containers to form a 0.5 - 10 mm thick layer of culture;
4) subject the culture to a UV treatment,
5) culture the mutated cells to obtain a culture of exponential growing cells,
6) transfer an aliquot of bacteria to one or more petridishes containing a suitable agar growth medium, the aliquot of bacteria are selected to give single colonies,
7) identify those colonies that have acquired antibiotic sensitivity by replica plating to petridishes with and without tetracycline antibiotic, and
8) isolate, expand and keep those tetracycline antibiotic sensitive colonies identified as a new tetracycline antibiotic sensitive strain; and
wherein the Minimum inhibitive Concentration (MIC) of tetracycline of the progenitor strain is at least 10 microgram tetracycline/ml and the MIC of tetracycline of the antibiotic sensitive strain is 1.5 µg tetracycline/ml or less.

2. The method according to claim 1, wherein the culture subsequent to step 4) is subjected to an enrichment step for mutations comprising the steps of:
4a) transfer an aliquot of the UV treated culture to fresh medium containing a dose of a penicillin analogue which is detrimental to exponentially growing cells, but tolerable to non-growing cells, and
4b) culture the cells in said penicillin analogue comprising medium under conditions which would promote exponential growth in the absence of penicillin or an analogue of penicillin such as ampicillin.

3. A *Bifidobacterium* strain which is sensitive to tetracyclines due to an inactivating mutation in *tetW* located on the chromosome, wherein the *tetW* gene comprises at least one sequence selected from the group of SEQ ID 3 [GGA TAC TGA ACC] and SEQ ID NO: 6 [GTG GTT TAG TCT]; and wherein the Minimum inhibitive Concentration (MIC) of tetracycline of the sensitive strain is 1.5 µg tetracycline/ml or less.

4. The *Bifidobacterium* strain according to claim 3, which is identified as *Bifidobacterium animalis* subspecies *lactis* strain CHCC8902 (Bb-12Tet-S139) and deposited on April 28, 2005 with the Deutsche Sammlung von Mikroorganismen und Zellkulturen under accession number DSM17281.

5. The *Bifidobacterium* strain according to claim 3, which is identified as *Bifidobacterium animalis* subspecies *lactis* strain CHCC9070 (DR10Tet-S9X) and deposited on April 28, 2005 with the Deutsche Sammlung von Mikroorganismen und Zellkulturen under accession number DSM17282.

6. A *Bifidobacterium animalis* subspecies *lactis* strain, which is sensitive to tetracycline due to an inactivating mutation in *tetW* located on the chromosome, and wherein the Minimum inhibitive Concentration (MIC) of tetracycline of the sensitive strain is 1.5 µg tetracycline/ml or less.

7. The *Bifidobacterium animalis* subspecies *lactis* strain of claim 6, wherein the progenitor strain is Bb-12 or HN019 (DR10).

8. Use of a *Bifidobacterium* strain of any of claims 3 to 7 for the preparation of an ingestible material or a bacterial culture.

9. Use of an ingestible material or a bacterial culture of claim. 8 that comprises a *Bifidobacterium* strain of any of claims 3-7, wherein the ingestible material or a bacterial culture is used for the preparation of a composition for the treatment or prevention of a disease, syndrome or condition, or for the preparation of a composition for improving digestion of nutrients, or for the preparation of a composition for improving the general health status of a human being or a vertebrate animal.

## Patentansprüche

1. Verfahren zur Isolierung eines Bifidobacterium-Stamms, der ein mutiertes tetW auf seinem Chromosom enthält, wobei diese Mutation den Stamm empfindlich gegen Tetracycline macht, und besagter Stamm wird aus einem Tetracyclin-resistenten bakteriellen Progenitorstamm isoliert, bei dem der gegen Antibiotika resistente Phänotyp durch die Expression von tetW hervorgerufen wird, das stabil in seinem Chromosom integriert ist, wobei besagtes Verfahren umfasst, dass die Zellen einem chemischen Mutagen und einem physikalischen Mutagen ausgesetzt werden, und
wobei das Verfahren folgende Phasen umfasst:
1) Züchtung der Progenitorzellen, um eine Kultur von exponentiell wachsenden Zellen zu erhalten,
2) Übertragung eines Teils der Zellen in ein frisches Medium, das Ethidiumbromid (EtBr) enthält,
3) Übertragung der Kultur in einen oder mehrere Behälter, um eine 0.5 — 10 mm dicke Schicht der Kultur zu bilden,
4) UV-Behandlung der Kultur,
5) Züchtung der mutierten Zellen, um eine exponentiell wachsende Zellkultur zu erhalten,
6) Übertragung eines Teils der Bakterien in eine oder mehrere Petrischalen, die ein passendes Agarwachstumsmedium enthalten; der Teil der Bakterien wird so ausgewählt, dass einzelne Kolonien entstehen,
7) Identifizierung derjenigen Kolonien, die durch Replica-Plating in Petrischalen mit und ohne Tetracyclinantibiotikum Empfindlichkeit gegen Antibiotika erworben haben, und
8) Isolierung, Erweiterung und Erhalten jener gegen Antibiotika empfindlichen Tetracyclinkolonien, die als ein neuer gegen Antibiotika empfindlicher Tetracyclinstamm identifiziert werden;
und bei dem die Minimale Hemm-Konzentration (MHK) von Tetracyclin des Progenitorstammes mindestens 10 Mikrogramm Tetracyclin/ml und die MHK des Tetracyclins-des gegen Antibiotika empfindlichen Stammes 1.5 µg Tetracyclin/ml oder weniger beträgt.

2. Verfahren nach Anspruch 1, bei dem die Kultur nach Phase 4) einer Anreicherungsphase für Mutationen mit folgenden Phasen ausgesetzt wird:
4a) Übertragung eines Teils der UV-behandelten Kultur in ein frisches Medium, das eine Dosis eines Penizillin-Analogons enthält, das schädlich für exponentiell wachsende Zellen ist, aber von nicht wachsenden Zellen vertragen wird, und
4b) Züchtung der Zellen in besagtem ein Penizillin-Analogon enthaltenden Medium unter Bedingungen, die das exponentielle Wachstum unter Ausschluss von Penizillin oder eines Penizillin-Analogons wie zum Beispiel Ampicillin fördern.

3. Bifidobacterium-Stamm, der aufgrund einer inaktivierenden Mutation in tetW auf dem Chromosom empfindlich gegen Tetracycline ist, wobei das tetW-Gen mindestens eine Sequenz ausgewählt aus der Gruppe SEQ ID 3 [GGA TAC TGA ACC] und SEQ ID NO: 6 [GTG GTT TAG TCT] umfasst; und bei dem die Minimale Hemm-Konzentration (MHK) von Tetracyclin des empfindlichen Stammes 1.5 µg Tetracyclin/ml oder weniger beträgt.

4. Bifidobacterium-Stamm nach Anspruch 3, der als Bifidobacterium animalis subspecies lactis-Stamm CHCC8902 (Bb-12Tet-S139) identifiziert ist und am 28. April 2005 bei der Deutschen Sammlung von Mikroorganismen und Zellkulturen unter der Zugangsnummer DSM17281 hinterlegt wurde.

5. Bifidobacterium-Stamm nach Anspruch 3, der als Bifidobacterium animalis subspecies lactis-Stamm CHCC9070 (DR10Tet-S9X) identifiziert ist und am 28. April 2005 bei der Deutschen Sammlung von Mikroorganismen und Zellkulturen unter der Zugangsnummer DSM17282 hinterlegt wurde.

6. Bifidobacterium animalis subspecies lactis-Stamm, der aufgrund einer inaktivierenden Mutation in tetW auf dem Chromosom empfindlich gegen Tetracyclin ist, und bei dem die minimale Hemm-Konzentration (MHK) von Tetracyclin des empfindlichen Stammes 1.5 µg Tetracyclin/ml oder weniger beträgt.

7. Bifidobacterium animalis subspecies lactis-Stamm nach Anspruch 6, bei dem der Progenitorstamm Bb-12 oder HN019 (DR10) ist.

8. Verwendung eines Bifidobacterium-Stammes nach einem der Ansprüche 3 bis 7 zur Herstellung eines essbaren Materials oder einer Bakterienkultur.

9. Verwendung eines essbaren Materials oder einer Bakterienkultur nach Anspruch 8, das/die einen Bifidobacterium-Stamm nach einem der Ansprüche 3-7 umfasst, wobei das essbare Material oder ein Bakterienkulturmaterial zur Herstellung einer Zusammensetzung zur Behandlung oder Vorbeugung einer Krankheit, eines Syndroms oder Zustands benutzt wird, oder zur Verbesserung der Nährstoffverdauung, oder um den allgemeinen Gesundheitszustand eines Menschen oder eines Wirbeltieres zu verbessern.

## Revendications

1. Procédé pour isoler une souche de *Bifidobacterium* contenant un *tetW* muté sur son chromosome, ladite mutation rend la souche sensible aux tétracyclines et ladite souche est isolée d'une souche bactérienne progénitrice résistante à la tétracycline où le phénotype antibiotique résistant est causé par l'expression du *tetW* stablement intégré en son chromosome, ledit procédé comprenant l'exposition des cellules à un mutagène chimique et un mutagène physique, et
où le procédé comprend les phases de:
1) cultiver les cellules progénitrices pour obtenir une culture de cellules à croissance exponentielle,
2) transférer une aliquote des cellules à un milieu frais contenant du bromure d'éthidium (EtBr),
3) transférer la culture à un ou plusieurs récipients pour former une couche de culture de 0.5 - 10 mm d'épaisseur,
4) soumettre la culture à un traitement UV,
5) cultiver les cellules mutées pour obtenir une culture de cellules à croissance exponentielle,
6) transférer une aliquote de bactéries à une ou plusieurs boîtes de Petri contenant un milieu de croissance d'agar approprié, l'aliquote de bactéries est sélectionnée pour donner des colonies individuelles,
7) identifier les colonies qui ont acquis une sensibilité antibiotique par la réplique à des boîtes de Petri avec et sans antibiotique de tétracycline, et
8) isoler, étendre et garder parmi les colonies sensibles à l'antibiotique de tétracycline celles qui ont été identifiées comme une nouvelle souche sensible à l'antibiotique de tétracycline; et
où la concentration minimale inhibitrice (CMI) de la tétracycline de la souche progénitrice est d'au moins 10 microgrammes/ml de tétracycline et la CMI de la tétracycline de la souche antibiotique sensible est 1.5 µg/ml de tétracycline ou moins.

2. La méthode selon la revendication 1, dans laquelle la culture après la phase
4) est soumise à une phase d'enrichissement pour des mutations, comprenant les phases de :
4a) transférer une aliquote de la culture traitée aux UV à un milieu frais contenant une dose d'un analogue de pénicilline qui est nuisible à des cellules à croissance exponentielle, mais tolérable à des cellules ne croissant pas, et
4b) cultiver les cellules dans ledit milieu comprenant un analogue de pénicilline sous des conditions qui promouvraient la croissance exponentielle en l'absence de pénicilline ou d'un analogue de la pénicilline comme l'ampicilline.

3. Une souche de *Bifidobacterium* qui est sensible aux tétracyclines à cause d'une mutation inactivante en *tetW* situé sur le chromosome, dans lequel le gène de *tetW* comprend au moins une séquence sélectionnée du groupe: SEQ ID 3 [GGA TAC TGA ACC] et SEQ ID NO: 6 [GTG GTT TAG TCT]; et dans laquelle la concentration active minimale (MIC) de la tétracycline de la souche sensible est 1.5 µg de tétracycline/ml ou moins.

4. La souche de *Bifidobacterium* selon la revendication 3, qui est identifiée comme souche de *Bifidobacterium animalis* sous-espèce *lactis* CHCC8902 (Bb-12Tet-S139) et a été déposée le 28 avril 2005 auprès du Deutsche Sammlung von Mikroorganismen und Zellkulturen sous le numéro d'entrée DSM17281.

5. La souche de *Bifidobacterium* selon la revendication 3, qui est identifiée comme souche de *Bifidobacterium animalis* sous-espèce *lactis* CHCC9070 (DR10Tet-S9X) et a été déposée le 28 avril 2005 auprès du Deutsche Sammlung von Mikroorganismen und Zellkulturen sous le numéro d'entrée DSM17282.

6. Une souche de *Bifidobacterium animalis* sous-espèce *lactis* qui est sensible à la tétracycline à cause d'une mutation inactivante en *tetW* situé sur le chromosome, et dans laquelle la concentration active minimale (MIC) de la tétracycline de la souche sensible est 1.5 µg de tétracycline/ml ou moins.

7. La souche de *Bifidobacterium animalis* sous-espèce *lactis* de la revendication 6, dans laquelle la souche progénitrice est Bb-12 ou HN019 (DR10).

8. Utilisation d'une souche de *Bifidobacterium* de l'une quelconque des revendications 3 à 7 pour la préparation d'une matière comestible ou d'une culture bactérienne.

9. Utilisation d'une matière comestible ou d'une culture bactérienne de la revendication 8 qui comprend une souche de *Bifidobacterium* de l'une quelconque des revendications 3-7, où la matière comestible ou une matière de culture bactérienne est utilisée pour la préparation d'une composition pour le traitement ou la prévention d'une maladie, d'un syndrome ou d'une condition, ou pour améliorer la digestion de substances nutritives, ou pour améliorer l'état de santé général d'un être humain ou d'un animal vertébré.


### SEQUENCE LISTING

<110> Chr. Hansen A/S
<120> NEW ANTIBIOTIC-SENSITIVE LACTIC ACID BACTERIA STRAINS
<130> P2195EP00
<160> 22
<170> PatentIn version 3.1
<210> 1
   <211> 24
   <212> DNA
   <213> Bifidobacterium animalis subsp. lactis.
<400> 1
   tcgctgggat acttgaacca gagt 24
<210> 2
   <211> 24
   <212> DNA
   <213> Bifidobacterium animalis subsp. lactis.
<400> 2
   tcgctgggat actgaaccag agtt 24
<210> 3
   <211> 12
   <212> DNA
   <213> Bifidobacterium animalis subsp. lactis
<400> 3
   ggatactgaa cc 12
<210> 4
   <211> 24
   <212> DNA
   <213> Bifidobacterium animalis subsp. lactis
<400> 4
   cagagcgtgg ttcagtctgt tcgg 24
<210> 5
   <211> 24
   <212> DNA
   <213> Bifidobacterium animalis subsp. lactis
<400> 5
   cagagcgtgg tttagtctgt tcgg 24
<210> 6
   <211> 12
   <212> DNA
   <213> Bifidobacterium animalis subsp. lactis
<400> 6
   gtggtttagt ct 12
<210> 7
   <211> 26
   <212> DNA
   <213> Artificial
<400> 7
   gagcatcgct taattccttc gagggg 26
<210> 8
   <211> 29
   <212> DNA
   <213> artificial
<400> 8
   cgaggggact ttggcccacc gctgggtgg 29
<210> 9
   <211> 25
   <212> DNA
   <213> Artificial
<400> 9
   gcaggcaggc acgtccatcc gcctc 25
<210> 10
   <211> 26
   <212> DNA
   <213> Artificial
<400> 10
   ggcttctgcc accgtggcct cgtcac 26
<210> 11
   <211> 27
   <212> DNA
   <213> Artificial
<400> 11
   cctgtccttg tccacgccga tatgcgc 27
<210> 12
   <211> 30
   <212> DNA
   <213> Artificial
<400> 12
   cggcacctgc tgtataatgc ggattgtggc 30
<210> 13
   <211> 27
   <212> DNA
   <213> Artificial
<400> 13
   gtgggaacaa aggattatga tagtccc 27
<210> 14
   <211> 26
   <212> DNA
   <213> Artificial
<400> 14
   ggctggcgtt gatttgcaga gcgtgg 26
<210> 15
   <211> 23
   <212> DNA
   <213> Artificial
<400> 15
   gcagacggtg tcgctgtccc cgg 23
<210> 16
   <211> 23
   <212> DNA
   <213> Artificial
<400> 16
   ccggggacag cgacaccgtc tgc 23
<210> 17
   <211> 26
   <212> DNA
   <213> Artificial
<400> 17
   ggagcggccg ctcaaagcag ccagcc 26
<210> 18
   <211> 26
   <212> DNA
   <213> Artificial
<400> 18
   ggctggctgc tttgagcggc cgctcc 26
<210> 19
   <211> 27
   <212> DNA
   <213> Artificial
<400> 19
   gcccaaaacg ggttgggcgg cacctcg 27
<210> 20
   <211> 24
   <212> DNA
   <213> Artificial
<400> 20
   ccagcccgta acggatacca tccc 24
<210> 21
<211> 30
   <212> DNA
   <213> Artificial
<400> 21
   gatggtcgca tgatcggcgg ggtcactccc 30
<210> 22
   <211> 3240
   <212> DNA
   <213> Bifidobacterium animalis subsp lactis.
<400> 22
   cagatgacga tttccgcatt cagcgacgaa ctggcacagg tgcggacgaa gaaaaaagca     60
   tttctcgacc agattgaacg gatcgtcccg tggaaggaat ggcttgccat gattcagccg    120
   tgctattaca aaggagagcg cggcaacaaa ccctatccgc tggagatcat gctccgactg    180
   tatctgctgc aaaacctcta tgacctgagt gacgaggcca cggtggcaga agccatcgac    240
   agccgcgcat tttcggagtt ctgcggcgtc gattccagca accaggttcc gaacggggat    300
   actcttggcc ggttccggaa cttgctgatc aagaacggac tgcaggagaa gctgttcgct    360
   caggtggtag cagcgctcat ggaacgtggc ctcattctga aaaagggcac cattgtagat    420
   tccaccatca tttccgcccc ctcttctacc aagaataagg aaaagaaacg ggatccggat    480
   gcccaccaag tcaagaaggg caacacctgg cactttgggt acaaagcgca tatcggcgtg    540
   gacaaggaca gcggactggt tcacacagtg gaagctacac cggcaaatgt ccacgacgtt    600
   gcggaagtgc cgaaattatt gacgggagag gaagaaacag tctatggaga cagcggttat    660
   ctcggcgcag gtaagcgcga agatgccgta gtccgaaaca aagctggccg gaaaatcaag    720
   tacaagatca atcgtcgtcc atcgcagatg aagaaactga gcaaaagcgg gcagtacgca    780
   gcaaagaaag cggaacgggc gaaatcctca gtgcgagcaa aagtagagca tgtattcggt    840
   gtcgttaaga agcagctgcg cttccgaaaa acgcgatacc gagggcttga aaagcaacaa    900
   gccaaattca atatcatgtt tgcgttggca aatctgattc tggctgacag accctgtctg    960
   gcagcttgag tcagtgcgcc tttgcggaca aaaaattcgg aggttatcca cagtttttat   1020
   tcggcacctg ctgtataatg cggattgtgg catttgtgcg gtgttgcctt aaataaaact   1080
   ataatcaaat agtgggaaca aaggattatg atagtccctt ttgtaggggc ttagtttttt   1140
   gtacccaatt taagaatact tttgccttat caattttgac atatccccaa aaacagcact 1200
   cacaaacagg tgtatgctgt atatgtgtat gtccgcaaat tatcatcccc agtggtaaaa 1260
   gtattttact gctggggatt tttatgccct tcggggcagt aaagggagga caatcacatg 1320
   aaaataatca atattggaat tcttgcccat gtagacgctg gaaagacgac cttgacggag 1380
   agcctgctat atgccagcgg agccatttca gaaccgggga gcgtcgaaaa agggacaacg 1440
   aggacggaca ccatgctttt ggagcggcag cgtgggatta ccattcaagc ggcagtcact 1500
   tccttccagt ggcacagatg taaagtcaac attgtggata cgcccggcca catggatttt 1560
   ttggcggagg tgtaccgctc tttggctgtt ttagatgggg ccatcttggt gatctccgct 1620
   aaagatggcg tgcaggccca gacccgtatt ctgttccatg ccctgcggaa aatgaacatt 1680
   cccaccgtta tctttatcaa caagatcgac caggctggcg ttgatttgca gagcgtggtt 1740
   cagtctgttc gggataagct ctccgccgat attatcatca agcagacggt gtcgctgtcc 1800
   ccggaaatag tcctggagga aaataccgac atagaagcat gggatgcggt catcgaaaat 1860
   aacgataaat tattggaaaa gtatatcgca ggagaaccaa tcagccggga aaaacttgtg 1920
   cgggaggaac agcggcgggt tcaagacgcc tccctgttcc cggtctatta tggcagcgcc 1980
   aaaaagggcc ttggcattca accgttgatg gatgcggtga cagggctgtt ccaaccgatt 2040
   ggggaacagg ggagcgccgc cctatgcggc agcgttttca aggtggagta tacagattgc 2100
   ggccagcggc gtgtctatct acggctatac agcggaacgc tgcgcctgcg ggatacggtg 2160
   gccctggccg ggagagaaaa gctgaaaatc acagagatgc gtattccatc caaaggggaa 2220
   attgttcgga cagacaccgc ttatccgggt gaaattgtta tccttcccag cgacagcgtg 2280
   aggttaaacg atgtattagg ggacccaacc cggctccctc gtaaaaggtg gcgtgaggac 2340
   cccctcccca tgctgcggac gtcgattgcg ccgaaaacgg cagcgcaaag agaacggctg 2400
   ctggacgctc ttacgcaact tgcggatact gacccgcttt tgcgctgcga ggtggattcc 2460
   atcacccatg agatcattct ttcttttttg ggccgggtgc agttggaggt tgtttccgct 2520
   ttgctgtcgg aaaaatacaa gcttgaaaca gtggtaaagg aacccaccgt catttatatg 2580
   gagcggccgc tcaaagcagc cagccacacc atccatatcg aggtgccgcc caacccgttt 2640
   tgggcatcca tcggactgtc tgttacacca ctcccgcttg gctccggtgt acaatacaag 2700
   agccgggttt cgctgggata cttgaaccag agttttcaaa acgctgtcag ggatggtatc 2760
   cgttacgggc tggagcaggg cttgttcggc tggaacgtaa cggactgtaa gatttgcttt 2820
   gaatacgggc tttattacag tccggtcagc acgccggcgg acttccgctc attggccccg 2880
   attgtattgg aacaggcatt gaaggaatca gggacgcaac tgctggaacc ttatctctcc 2940
   ttcaccctct atgcgccccg ggaatatctt tccagggctt atcatgatgc accgaaatac   3000
   tgtgccacca tcgaaacggt ccaggtaaaa aaggatgaag ttgtctttac tggcgagatt   3060
   cccgcccgct gtatacaggc ataccgtact gatctggcct tttacaccaa cgggcagagc   3120
   gtatgcctta cagaactgaa agggtatcag gccgctgtcg gcaagccagt catccagccc   3180
   cgccgtccaa acagccgcct ggacaaggtg cgccatatgt tcagtaagat cacttgatac   3240